# EUROPEAN PATENT APPLICATION

(11) **EP 3 517 522 A1**
(43) Date of publication of application: **31.07.2019**
(21) Application number: 17853047.3
(22) Date of filing: 20.09.2017
(51) Int. Cl.: C07C 39/15, C07C 39/17, G03F 7/11, G03F 7/20, G03F 7/40, H01L 21/027

(54) **COMPOUND, RESIN, COMPOSITION, RESIST PATTERN FORMING METHOD AND CIRCUIT PATTERN FORMING METHOD**

(30) Priority: 20.09.2016 JP 2016183026
(71) Applicant: Mitsubishi Gas Chemical Company, Inc., Tokyo 100-8324 (JP)
(72) Inventor: ECHIGO, Masatoshi, Tokyo 100-8324 (JP)
(74) Representative: Müller-Boré & Partner Patentanwälte PartG mbB
(86) International application number: PCT/JP2017/033799
(87) International publication number: WO 2018/056277

(57) **Abstract**

The present invention provides a compound represented by the following formula (0): 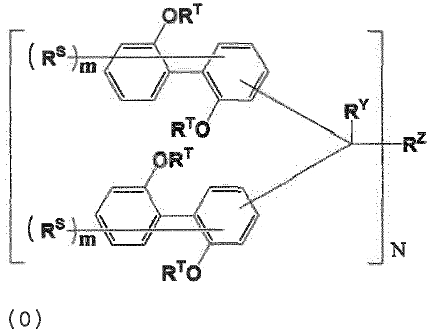 wherein R^{Y} is a hydrogen atom, an alkyl group, which has 1 to 30 carbon atoms or an aryl group, which has 6 to 30 carbon atoms;
R^{Z} is an N-valent group, which has 1 to 60 carbon atoms or a single bond;
R^{T} is a hydrogen atom;
each R^{S} is independently an alkyl group, which has 1 to 30 carbon atoms, and which optionally has a substituent, an aryl group, which has 6 to 30 carbon atoms, and which optionally has a substituent, an alkenyl group, which has 2 to 30 carbon atoms, and which optionally has a substituent, an alkoxy group, which has 1 to 30 carbon atoms, and which optionally has a substituent, a halogen atom, a nitro group, an amino group, a carboxylic acid group, a thiol group or a hydroxy group, wherein the alkyl group, the aryl group, the alkenyl group, and the alkoxy group each optionally contain an ether bond, a ketone bond, or an ester bond;
each m is independently an integer of 0 to 7; and
N is an integer of 1 to 4, wherein when N is an integer of 2 or larger, N structural formulas within the parentheses [ ] are the same or different.

## Description

### Technical Field

The present invention relates to a compound, a resin and a composition having a specific structure, a resist pattern formation method and a circuit pattern formation method.

### Background Art

In the production of semiconductor devices, fine processing is practiced by lithography using photoresist materials. In recent years, further miniaturization based on pattern rules has been demanded along with increase in the integration and speed of LSI. The light source for lithography used upon forming resist patterns has been shifted to ArF excimer laser (193 nm) having a shorter wavelength from KrF excimer laser (248 nm). The introduction of extreme ultraviolet (EUV, 13.5 nm) is also expected.

However, because conventional polymer-based resist materials have a molecular weight as large as about 10,000 to 100,000 and also wide molecular weight distribution, in lithography using such a polymer-based resist material, roughness occurs on a pattern surface; the pattern dimension becomes difficult to be controlled; and there is a limitation in miniaturization. Accordingly, various low molecular weight resist materials have been proposed so far in order to provide resist patterns having higher resolution. The low molecular weight resist materials are expected to provide resist patterns having high resolution and small roughness, because of their small molecular sizes.

Various materials are currently known as such low molecular resist materials. For example, an alkaline development type negative type radiation-sensitive composition (see, for example, Patent Literature 1 and Patent Literature 2) using a low molecular weight polynuclear polyphenolic compound as a main component has been suggested; and as a candidate of a low molecular weight resist material having high heat resistance, an alkaline development type negative type radiation-sensitive composition (see, for example, Patent Literature 3 and Non Patent Literature 1) using a low molecular weight cyclic polyphenolic compound as a main component has been suggested as well. Also, as a base compound of a resist material, a polyphenol compound is known to be capable of imparting high heat resistance despite a low molecular weight and useful for improving the resolution and roughness of a resist pattern (see, for example, Non Patent Literature 2).

The present inventors have proposed so far a resist composition containing a compound having a specific structure and an organic solvent (see e.g., Patent Literature 4) as a material that is excellent in etching resistance and is also soluble in a solvent and applicable to a wet process.

Also, the light source for lithography used upon forming resist patterns has been shifted to ArF excimer laser (193 nm) having a shorter wavelength from KrF excimer laser (248 nm). However, as the miniaturization of resist patterns proceeds, the problem of resolution or the problem of collapse of resist patterns after development arises. Therefore, resists have been desired to have a thinner film. However, if resists merely have a thinner film, it is difficult to obtain the film thicknesses of resist patterns sufficient for substrate processing. Therefore, there has been a need for a process of preparing a resist underlayer film between a resist and a semiconductor substrate to be processed, and imparting functions as a mask for substrate processing to this resist underlayer film in addition to a resist pattern.

Various resist underlayer films for such a process are currently known. For example, in order to achieve a resist underlayer film for lithography having the selectivity of a dry etching rate close to that of resists, unlike conventional resist underlayer films having a fast etching rate, an underlayer film forming material for a multilayer resist process containing a resin component having at least a substituent that generates a sulfonic acid residue by eliminating a terminal group under application of predetermined energy, and a solvent has been suggested (see e.g., Patent Literature 5). Also, in order to achieve a resist underlayer film for lithography having the selectivity of a dry etching rate smaller than that of resists, a resist underlayer film material comprising a polymer having a specific repeat unit has been suggested (see e.g., Patent Literature 6). Furthermore, in order to achieve a resist underlayer film for lithography having the selectivity of a dry etching rate smaller than that of semiconductor substrates, a resist underlayer film material comprising a polymer prepared by copolymerizing a repeat unit of an acenaphthylene and a repeat unit having a substituted or unsubstituted hydroxy group has been suggested (see e.g., Patent Literature 7).

Meanwhile, as materials having high etching resistance for this kind of resist underlayer film, amorphous carbon underlayer films formed by CVD using methane gas, ethane gas, acetylene gas, or the like as a raw material are well known. However, resist underlayer film materials that can form resist underlayer films by a wet process such as spin coating or screen printing have been demanded from the viewpoint of a process.

The present inventors have proposed an underlayer film forming composition for lithography containing a compound having a specific structure and an organic solvent (see e.g., Patent Literature 8) as a material that is excellent in etching resistance, has high heat resistance, and is soluble in a solvent and applicable to a wet process.

As for methods for forming an intermediate layer used in the formation of a resist underlayer film in a three-layer process, for example, a method for forming a silicon nitride film (see e.g., Patent Literature 9) and a CVD formation method for a silicon nitride film (see e.g., Patent Literature 10) are known. Also, as intermediate layer materials for a three-layer process, materials comprising a silsesquioxane-based silicon compound are known (see e.g., Patent Literature 11 and Patent Literature 12).

Various compositions have also been proposed as optical component forming compositions. Examples thereof include acrylic resins (see, for example, Patent Literatures 13 and 14) and polyphenol having a specific structure derived from an allyl group (see, for example, Patent Literature 15).

### Citation List

### Patent Literature

Patent Literature 1: Japanese Patent Laid-Open No. 2005-326838
Patent Literature 2: Japanese Patent Laid-Open No. 2008-145539
Patent Literature 3: Japanese Patent Laid-Open No. 2009-173623
Patent Literature 4: International Publication No. WO 2013/024778
Patent Literature 5: Japanese Patent Laid-Open No. 2004-177668
Patent Literature 6: Japanese Patent Laid-Open No. 2004-271838
Patent Literature 7: Japanese Patent Laid-Open No. 2005-250434
Patent Literature 8: International Publication No. WO 2013/024779
Patent Literature 9: Japanese Patent Laid-Open No. 2002-334869
Patent Literature 10: International Publication No. WO 2004/066377
Patent Literature 11: Japanese Patent Laid-Open No. 2007-226170
Patent Literature 12: Japanese Patent Laid-Open No. 2007-226204
Patent Literature 13: Japanese Patent Laid-Open No. 2010-138393
Patent Literature 14: Japanese Patent Laid-Open No. 2015-174877
Patent Literature 15: International Publication No. WO 2014/123005

### Non Patent Literature

Non Patent Literature 1: T. Nakayama, M. Nomura, K. Haga, M. Ueda: Bull. Chem. Soc. Jpn., 71, 2979 (1998)
Non Patent Literature 2: Shinji Okazaki et al., "New Trends of Photoresists", CMC Publishing Co., Ltd., September 2009, pp. 211-259

### Summary of Invention

### Technical Problem

As mentioned above, a large number of film forming compositions for lithography for resist purposes and film forming compositions for lithography for underlayer film purposes have heretofore been suggested. However, none of these compositions not only have high solvent solubility that permits application of a wet process such as spin coating or screen printing but achieve both of heat resistance and etching resistance at high dimensions. Thus, the development of novel materials is required.

Also, a large number of compositions for optical members have heretofore been suggested. However, none of these compositions achieve all of heat resistance, transparency, and refractive index at high dimensions. Thus, the development of novel materials is required.

The present invention has been made in light of the problems of the conventional techniques mentioned above. An object of the present invention is to provide a compound, a resin, and a composition that are applicable to a wet process and are useful for forming a photoresist and an underlayer film for photoresists excellent in heat resistance, solubility, and etching resistance.

### Solution to Problem

The inventors have, as a result of devoted examinations to solve the problems of the conventional techniques described above, found out that a compound or a resin having a specific structure can solve the problems of the conventional techniques described above, and reached the present invention.

More specifically, the present invention is as follows.
[1] A compound represented by following formula (0): 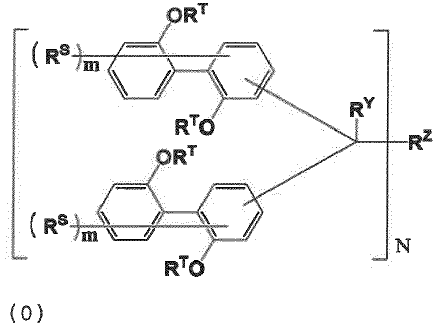
   wherein R^{Y} is a hydrogen atom, an alkyl group, which has 1 to 30 carbon atoms or an aryl group, which has 6 to 30 carbon atoms;
   R^{Z} is an N-valent group, which has 1 to 60 carbon atoms or a single bond;
   R^{T} is a hydrogen atom;
   each R^{S} is independently an alkyl group, which has 1 to 30 carbon atoms, and which optionally has a substituent, an aryl group, which has 6 to 30 carbon atoms, and which optionally has a substituent, an alkenyl group, which has 2 to 30 carbon atoms, and which optionally has a substituent, an alkoxy group, which has 1 to 30 carbon atoms, and which optionally has a substituent, a halogen atom, a nitro group, an amino group, a carboxylic acid group, a thiol group or a hydroxy group, wherein the alkyl group, the aryl group, the alkenyl group, and the alkoxy group each optionally contain an ether bond, a ketone bond, or an ester bond;
   each m is independently an integer of 0 to 7; and
   N is an integer of 1 to 4, wherein when N is an integer of 2 or larger, N structural formulas within the parentheses [ ] are the same or different.
[2] The compound according to [1], wherein the compound represented by the above formula (0) is a compound represented by following formula (1): 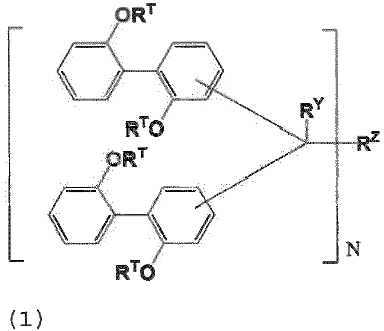 wherein R^{Y}, R^{Z}, R^{T} and N are as defined above.
[3] The compound according to [2], wherein the compound represented by the above formula (1) is a compound represented by following formula (1-1): 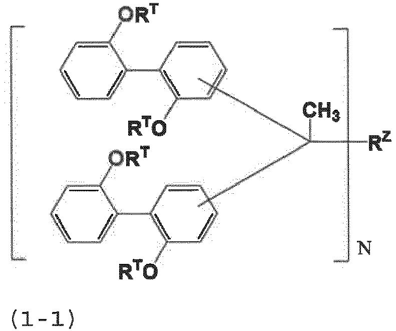 wherein R^{Z}, R^{T} and N are as defined above.
[4] The compound according to [2], wherein the compound represented by the above formula (1) is a compound represented by following formula (1-2): 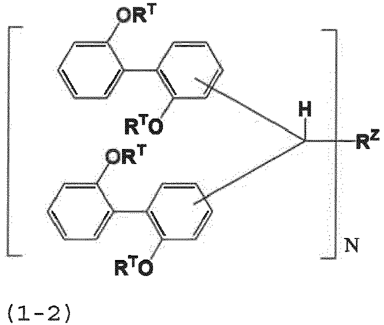 wherein R^{Y}, R^{Z}, R^{T} and N are as defined above.
[5] A resin obtained with the compound according to [1] as a monomer.
[6] The resin according to [5], wherein the resin has a structure represented by following formula (2): 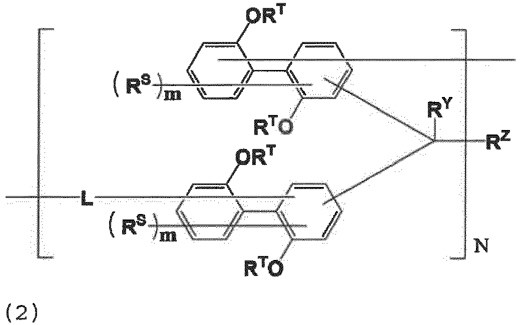
   wherein R^{Y}, R^{Z}, R^{T}, R^{S}, N and m are as defined above; and
   L is a linear, branched, or cyclic alkylene group, which has 1 to 30 carbon atoms, and which optionally has a substituent, an arylene group, which has 6 to 30 carbon atoms, and which optionally has a substituent, an alkoxylene group, which has 1 to 30 carbon atoms, and which optionally has a substituent or a single bond, wherein the alkylene group, the arylene group, and the alkoxylene group each optionally contain an ether bond, a ketone bond, or an ester bond.
[7] The resin according to [6], wherein the resin represented by the above formula (2) has a structure represented by following formula (3): 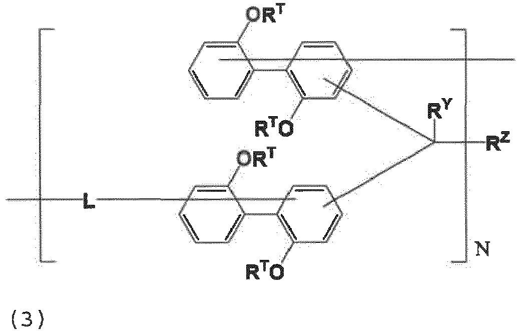 wherein R^{Y}, R^{Z}, R^{T}, N and L are as defined above.
[8] A composition comprising one or more selected from the group consisting of the compound according to any of [1] to [4] and the resin according to any of [5] and [6].
[9] The composition according to [8], further comprising a solvent.
[10] The composition according to [8] or [9], further comprising an acid generating agent.
[11] The composition according to any one of [8] to [10], further comprising a crosslinking agent.
[12] The composition according to [11], wherein the crosslinking agent is at least one selected from the group consisting of a phenol compound, an epoxy compound, a cyanate compound, an amino compound, a benzoxazine compound, a melamine compound, a guanamine compound, a glycoluril compound, a urea compound, an isocyanate compound and an azide compound.
[13] The composition according to [11] or [12], wherein the crosslinking agent has at least one allyl group.
[14] The composition according to any of [11] to [13], wherein a content of the crosslinking agent is 0.1 to 50% by mass based on a total mass of a solid component.
[15] The composition according to any of [11] to [14], further comprising a crosslinking promoting agent.
[16] The composition according to [15], wherein the crosslinking promoting agent is at least one selected from the group consisting of an amine, an imidazole, an organic phosphine, and a Lewis acid.
[17] The composition according to [15] or [16], wherein a content of the crosslinking promoting agent is 0.1 to 5% by mass based on a total mass of a solid component.
[18] The composition according to any of [8] to [17], further comprising a radical polymerization initiator.
[19] The composition according to any of [8] to [18], wherein the radical polymerization initiator is at least one selected from the group consisting of a ketone-based photopolymerization initiator, an organic peroxide-based polymerization initiator and an azo-based polymerization initiator.
[20] The composition according to any of [8] to [19], wherein a content of the radical polymerization initiator is 0.05 to 25% by mass based on a total mass of a solid component.
[21] The composition according to any of [8] to [20], wherein the composition is used in film formation for lithography.
[22] The composition according to any of [8] to [20], wherein the composition is used in resist permanent film formation.
[23] The composition according to any of [8] to [20], wherein the composition is an optical component forming composition.
[24] A method for forming a resist pattern, comprising the steps of: forming a photoresist layer on a substrate using the composition according to [21]; and then irradiating a predetermined region of the photoresist layer with radiation for development.
[25] A method for forming a resist pattern, comprising the steps of: forming an underlayer film on a substrate using the composition according to [21]; forming at least one photoresist layer on the underlayer film; and then irradiating a predetermined region of the photoresist layer with radiation for development.
[26] A method for forming a circuit pattern, comprising the steps of: forming an underlayer film on a substrate using the composition according to [21], forming an intermediate layer film on the underlayer film using a resist intermediate layer film material, and forming at least one photoresist layer on the intermediate layer film;
   irradiating a predetermined region of the photoresist layer with radiation for development, thereby forming a resist pattern; and
   etching the intermediate layer film with the resist pattern as a mask, etching the underlayer film with the obtained intermediate layer film pattern as an etching mask, and etching the substrate with the obtained underlayer film pattern as an etching mask, thereby forming a pattern on the substrate.

### Advantageous Effects of Invention

The compound and the resin according to the present invention have high solubility in a safe solvent and have good heat resistance and etching resistance. Furthermore, the resist composition comprising the compound and/or the resin according to the present invention imparts a good shape to a resist pattern.

### Description of Embodiments

Hereinafter, embodiments of the present invention will be described. The embodiments described below are given merely for illustrating the present invention. The present invention is not limited only by these embodiments.

The compound, the resin, and the composition comprising the compound and/or the resin of the present embodiment are applicable to a wet process and are useful for forming a photoresist underlayer film excellent in heat resistance and etching resistance. Furthermore, the composition of the present embodiment employs the compound or the resin having high heat resistance and also high solvent solubility and having a specific structure and can therefore form a resist and an underlayer film that is prevented from deteriorating during high temperature baking and is also excellent in etching resistance against oxygen plasma etching or the like. In addition, the underlayer film thus formed is also excellent in adhesiveness to a resist layer and can therefore form an excellent resist pattern.

Moreover, the composition of the present embodiment has high refractive index and is prevented from being stained by heat treatment in a wide range from a low temperature to a high temperature. Therefore, the composition is also useful as various optical forming compositions.

The compound and the resin of the present embodiment exhibit very low crystallinity. The composition comprising the compound and/or the resin is advantageous for forming a resist, an underlayer film, and an optical member.

### [Compound represented by formula (0)]

The compound of the present embodiment is represented by the following formula (0): 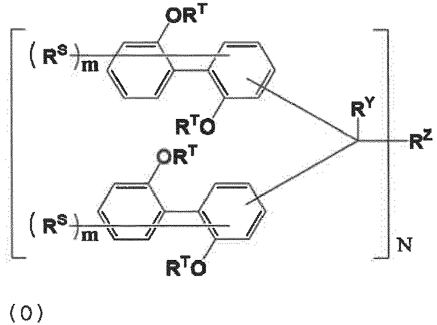
wherein R^{Y} is a hydrogen atom, an alkyl group, which has 1 to 30 carbon atoms or an aryl group, which has 6 to 30 carbon atoms;
R^{Z} is an N-valent group, which has 1 to 60 carbon atoms or a single bond;
R^{T} is a hydrogen atom;
each R^{S} is independently an alkyl group, which has 1 to 30 carbon atoms, and which optionally has a substituent, an aryl group, which has 6 to 30 carbon atoms, and which optionally has a substituent, an alkenyl group, which has 2 to 30 carbon atoms, and which optionally has a substituent, an alkoxy group, which has 1 to 30 carbon atoms, and which optionally has a substituent, a halogen atom, a nitro group, an amino group, a carboxylic acid group, a thiol group or a hydroxy group, wherein the alkyl group, the aryl group, the alkenyl group, and the alkoxy group each optionally contain an ether bond, a ketone bond, or an ester bond;
each m is independently an integer of 0 to 7; and N is an integer of 1 to 4, wherein when N is an integer of 2 or larger, N structural formulas within the parentheses [ ] are the same or different.
R^{Y} is a hydrogen atom, an alkyl group, which has 1 to 30 carbon atoms or an aryl group, which has 6 to 30 carbon atoms. A linear, branched, or cyclic alkyl group can be used as the alkyl group. When R^{Y} is a linear, branched, or cyclic alkyl group, which has 1 to 30 carbon atoms or an aryl group, which has 6 to 30 carbon atoms, excellent heat resistance and solvent solubility can be imparted.
R^{Z} is an N-valent group, which has 1 to 60 carbon atoms or a single bond, and each aromatic ring is bonded via this R^{Z}. N is an integer of 1 to 4. When N is an integer of 2 or larger, N structural formulas within the parentheses [ ] may be the same or different. The N-valent group refers to an alkyl group, which has 1 to 60 carbon atoms when N is 1, an alkylene group, which has 1 to 30 carbon atoms when N is 2, an alkanepropayl group, which has 2 to 60 carbon atoms when N is 3, and an alkanetetrayl group having 3 to 60 carbon atoms when N is 4. Examples of the N-valent group include groups having linear hydrocarbon groups, branched hydrocarbon groups, and alicyclic hydrocarbon groups. Herein, the alicyclic hydrocarbon groups also include bridged alicyclic hydrocarbon groups. Also, the N-valent hydrocarbon group may have an alicyclic hydrocarbon group, a double bond, a heteroatom, or an aromatic group, which has 6 to 60 carbon atoms.
R^{T} is a hydrogen atom.
Each R^{S} is independently an alkyl group, which has 1 to 30 carbon atoms, and which optionally has a substituent, an aryl group, which has 6 to 30 carbon atoms, and which optionally has a substituent, an alkenyl group, which has 2 to 30 carbon atoms, and which optionally has a substituent, an alkoxy group, which has 1 to 30 carbon atoms, and which optionally has a substituent, a halogen atom, a nitro group, an amino group, a carboxylic acid group, a thiol group or a hydroxy group, wherein the alkyl group, the aryl group, the alkenyl group, and the alkoxy group each optionally contain an ether bond, a ketone bond, or an ester bond. Each of the alkyl group, the alkenyl group, and the alkoxy group may be a linear, branched, or cyclic group.
Each m is independently an integer of 0 to 7.

Specific examples of the compound represented by the above formula (0) will be listed below. However, the compound represented by the formula (0) is not limited to the specific examples listed below.

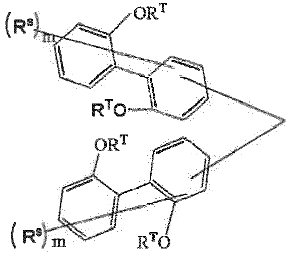

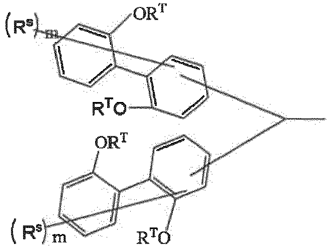

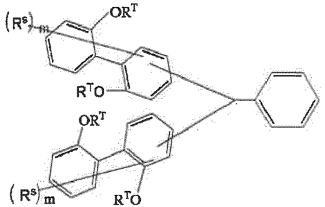

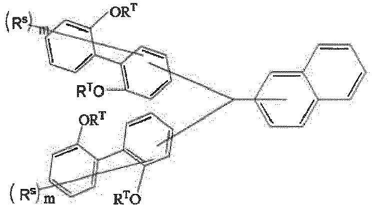

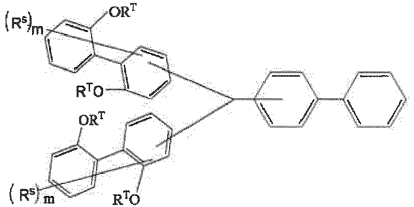

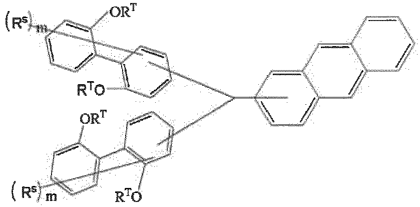

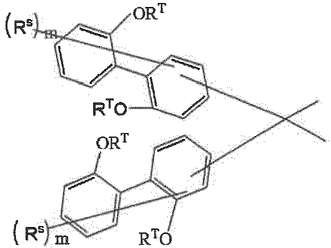

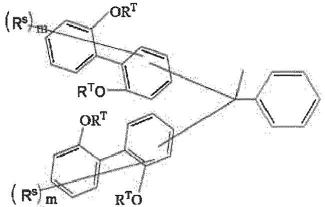

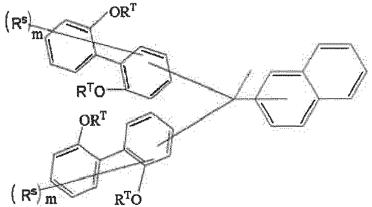

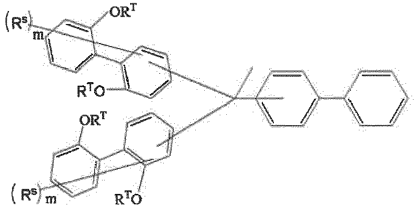

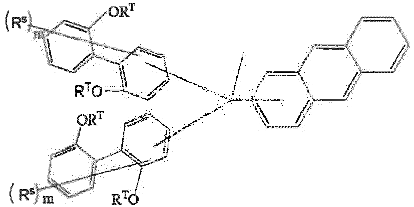

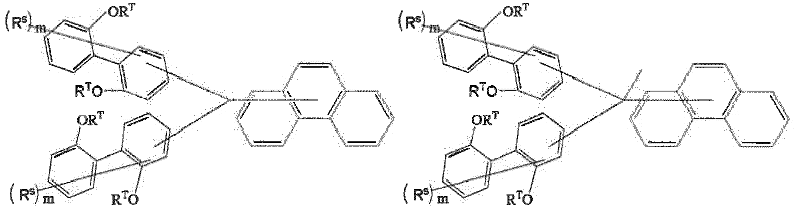

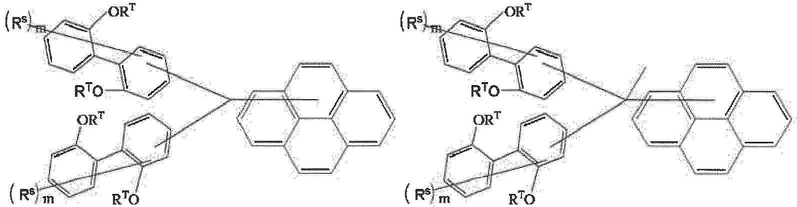

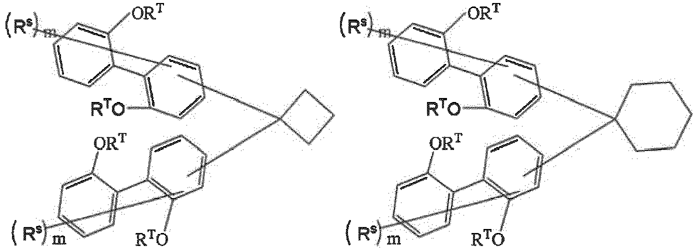

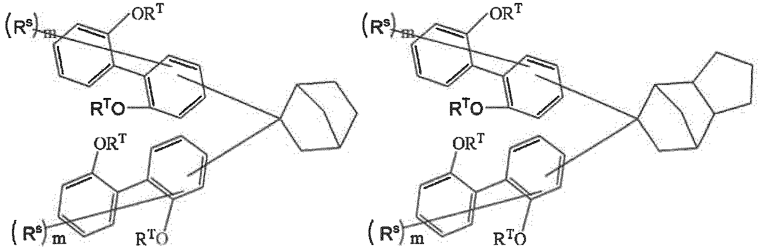

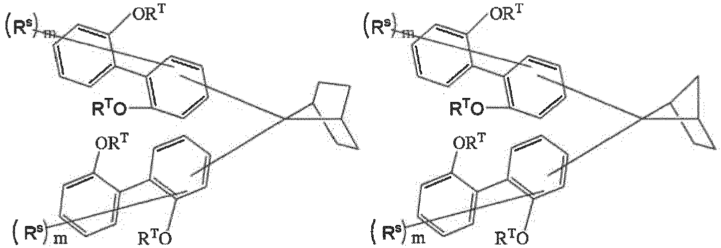

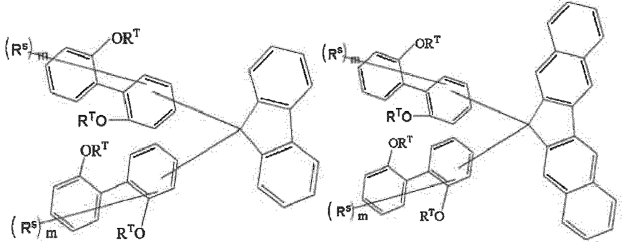

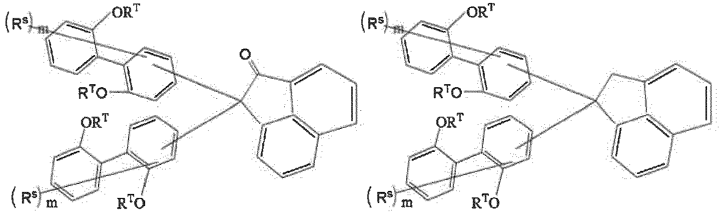

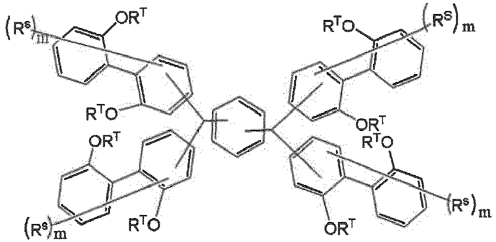

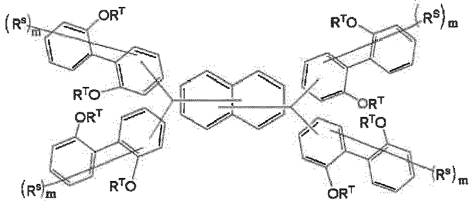

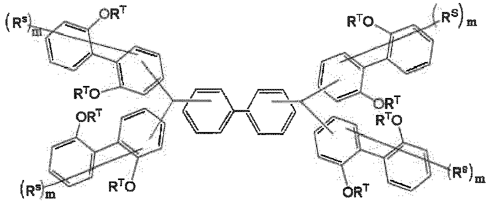

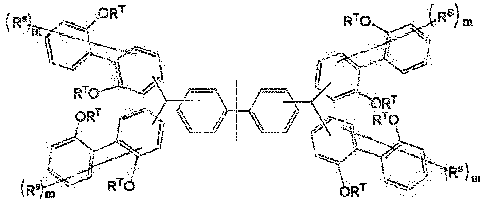

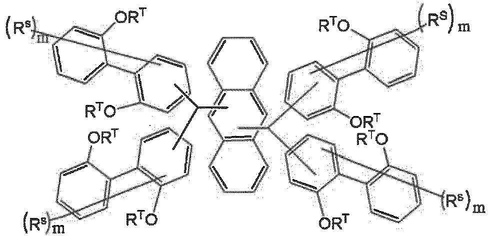

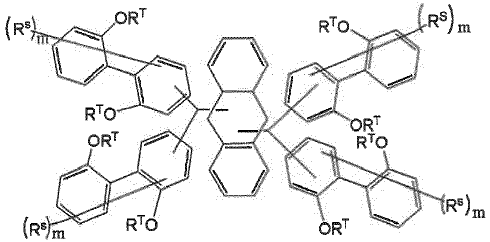

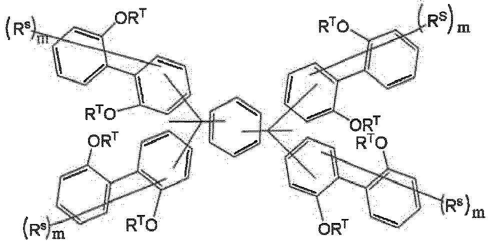

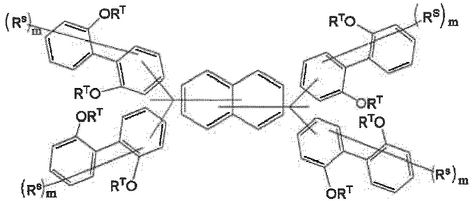

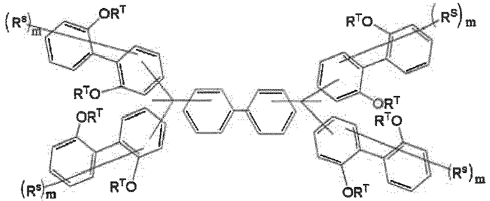

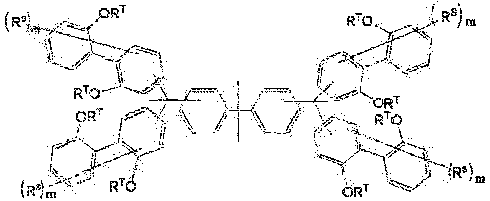

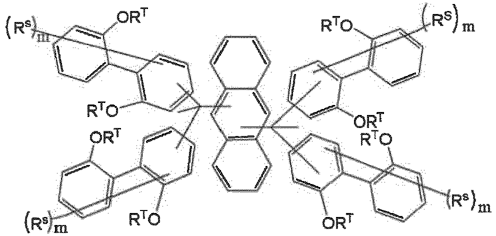

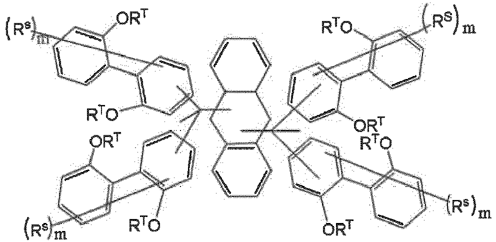

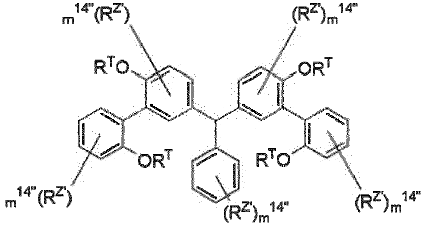

The compound represented by any of the above formulas is still more preferably a compound represented by any of the following formulas from the viewpoint of the availability of raw materials.

In the above formulas, R^{T} is as defined in the description of the above formula (0). R^{Z'} is as defined in R^{Z} in the above formula (0) . m¹⁴ is an integer of 0 to 5, m^{14'} is an integer of 0 to 4, and m^{14"} is an integer of 0 to 3.

Examples of R^{Z'} include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a phenyl group, a naphthyl group, an anthracene group, a pyrenyl group, a biphenyl group, a heptacene group, a vinyl group, an allyl group, a triacontenyl group, a methoxy group, an ethoxy group, a triacontyloxy group, a fluorine atom, a chlorine atom, a bromine atom, an iodine atom, and a thiol group.

R^{Z'} listed above includes isomers. For example, a butyl group includes a n-butyl group, an isobutyl group, a sec-butyl group, and a tert-butyl group.

### [Compound represented by formula (1)]

The compound (0) of the present embodiment is preferably a compound represented by the following formula (1) from the viewpoint of heat resistance and solvent solubility.

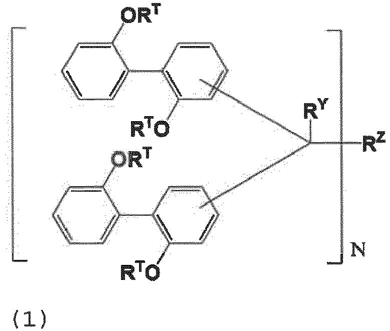

In the above formula (1), R^{Y}, R^{Z}, R^{T}, and N are as defined above.

The compound represented by the above formula (1) has high heat resistance attributed to its rigid structure, in spite of its relatively low molecular weight, and can therefore be used even under high temperature baking conditions. Also, the compound represented by the above formula (1) has tertiary carbon or quaternary carbon in the molecule, which inhibits crystallinity, and is thus suitably used as a film forming composition for lithography that can be used in film production for lithography.

Furthermore, the compound represented by the formula (1) has high solubility in a safe solvent and has good heat resistance and etching resistance. Therefore, the resist forming composition for lithography comprising the compound represented by the above formula (1) can impart a good shape to a resist pattern.

Moreover, the compound represented by the formula (1) has a relatively low molecular weight and a low viscosity and therefore facilitates enhancing film smoothness while uniformly and completely filling even the steps of an uneven substrate (particularly having fine space, hole pattern, etc.). As a result, an underlayer film forming composition for lithography containing this compound has good embedding and smoothing properties. Moreover, the compound has a relatively high carbon concentration and can therefore also impart high etching resistance.

In addition, the compound represented by the formula (1) has high refractive index ascribable to its high aromatic density and is prevented from being stained by heat treatment in a wide range from a low temperature to a high temperature. Therefore, the compound represented by the formula (1) is also useful as various optical component forming compositions. Among others, the compound preferably has quaternary carbon from the viewpoint of preventing the compound from being oxidatively decomposed and stained and improving heat resistance and solvent solubility. The optical component is useful as a component in the form of a film or a sheet as well as a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, or a photosensitive optical waveguide.

The compound represented by the above formula (1) is more preferably a compound represented by the following formula (1-1) from the viewpoint of heat resistance and solubility in an organic solvent.

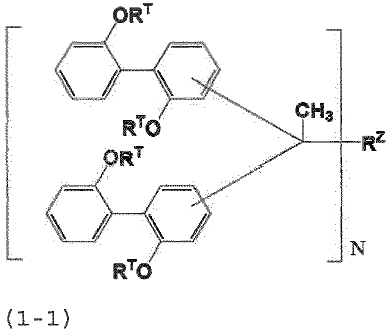

In the formula (1-1), R^{Z}, R^{T}, and N are as defined above.

The compound represented by the above formula (1) is also more preferably a compound represented by the following formula (1-2) from the viewpoint of easy crosslinking.

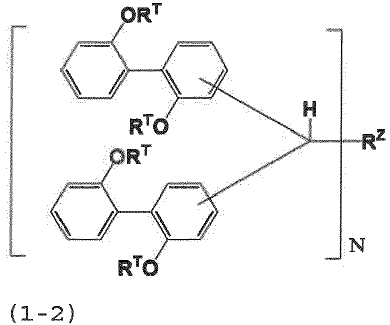

In the formula (1-2), R^{Y}, R^{Z}, R^{T}, and N are as defined above.

The compound represented by the above formula (1-1) is still more preferably a compound selected from the group consisting of compounds represented by the following formula (1a) from the viewpoint of the supply of raw materials.

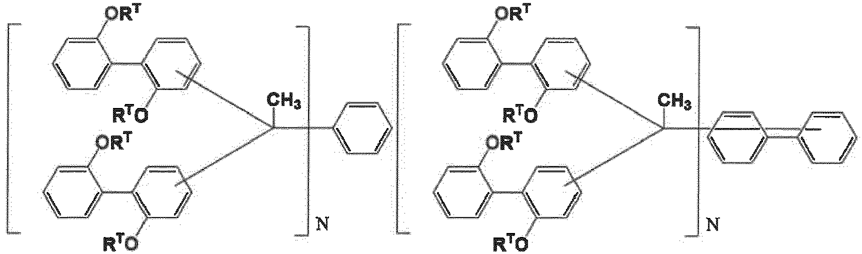

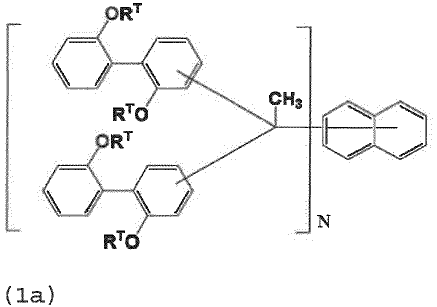

In the above formula (1a), R^{Y}, R^{Z}, R^{T}, and N are as defined in the description of the above formula (1).

The compound represented by the above formula (la) is further preferably a compound represented by any of the following formulas (BiF-1) and (BiF-2) from the viewpoint of further solubility in an organic solvent.

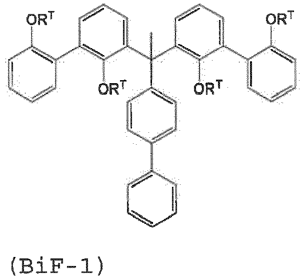

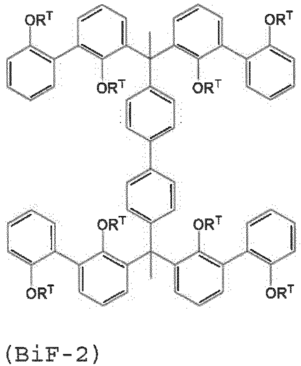

The compound represented by the above formula (1-2) is still more preferably a compound selected from the group consisting of compounds represented by the following formula (1b) from the viewpoint of the supply of raw materials.

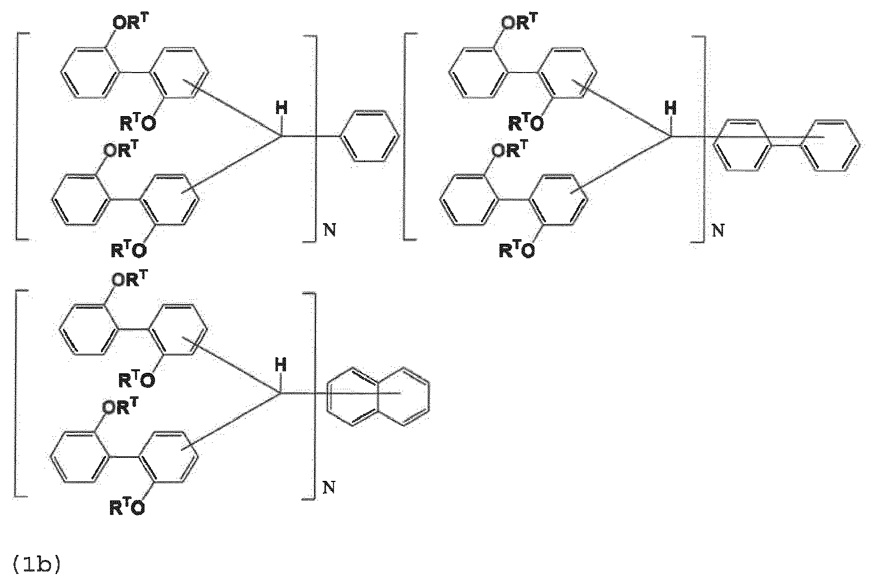

In the above formula (1a), R^{Y}, R^{Z}, R^{T}, and N are as defined in the description of the above formula (1).

The compound represented by the above formula (la) is further preferably a compound represented by any of the following formulas (BiF-3) and (BiF-4) from the viewpoint of further solubility in an organic solvent.

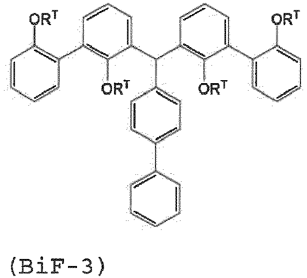

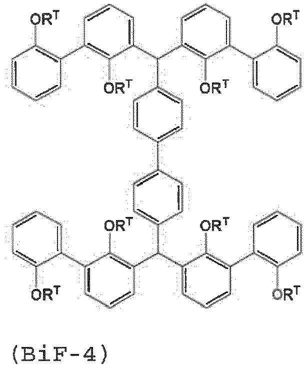

### [Method for producing compound represented by formula (0)]

The compound represented by the formula (0) of the present embodiment can be arbitrarily synthesized by the application of a publicly known approach, and the synthesis approach is not particularly limited.

A polyphenol compound can be obtained, for example, by subjecting a 2,2'-biphenol and a corresponding aldehyde or ketone to polycondensation reaction in the presence of an acid catalyst at normal pressure. If necessary, this reaction can also be carried out under increased pressure.

Examples of the 2,2'-biphenol include, but not particularly limited to, 2,2'-biphenol, 3,3'-dimethyl-2,2'-biphenol, and 3,3'-diphenyl-2,2'-biphenol. These 2,2'-biphenols can be used alone as one kind or can be used in combination of two or more kinds. Among them, 2,2'-biphenol is more preferably used from the viewpoint of the stable supply of raw materials.

Examples of the aldehyde include, but not particularly limited to, formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarbaldehyde, phenanthrenecarbaldehyde, pyrenecarbaldehyde, and furfural. These aldehydes can be used alone as one kind or can be used in combination of two or more kinds. Among them, benzaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, cyclohexylbenzaldehyde, biphenylaldehyde, naphthaldehyde, anthracenecarbaldehyde, phenanthrenecarbaldehyde, pyrenecarbaldehyde, or furfural is preferably used from the viewpoint of providing high heat resistance, and benzaldehyde, hydroxybenzaldehyde, chlorobenzaldehyde, nitrobenzaldehyde, methylbenzaldehyde, ethylbenzaldehyde, butylbenzaldehyde, cyclohexylbenzaldehyde, biphenylaldehyde, naphthaldehyde, anthracenecarbaldehyde, phenanthrenecarbaldehyde, pyrenecarbaldehyde, or furfural is more preferably used because of high etching resistance.

Examples of the ketone include, but not particularly limited to, acetone, methyl ethyl ketone, cyclobutanone, cyclopentanone, cyclohexanone, norbornanone, tricyclohexanone, tricyclodecanone, adamantanone, fluorenone, benzofluorenone, acenaphthenequinone, acenaphthenone, anthraquinone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, and diphenylcarbonylbiphenyl. These ketones can be used alone as one kind or can be used in combination of two or more kinds. Among them, cyclopentanone, cyclohexanone, norbornanone, tricyclohexanone, tricyclodecanone, adamantanone, fluorenone, benzofluorenone, acenaphthenequinone, acenaphthenone, anthraquinone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, or diphenylcarbonylbiphenyl is preferably used from the viewpoint of conferring high heat resistance, and acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, or diphenylcarbonylbiphenyl is more preferably used from the viewpoint of improving etching resistance.

As the ketone, a ketone having an aromatic ring is preferably used from the viewpoint that both high heat resistance and high etching resistance are achieved.

The acid catalyst used in the reaction can be arbitrarily selected and used from publicly known catalysts and is not particularly limited. Inorganic acids and organic acids are widely known as such acid catalysts, and examples include, but not particularly limited to, inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, organic acids and solid acids are more preferable from the viewpoint of production, and hydrochloric acid or sulfuric acid is preferably used from the viewpoint of production such as easy availability and handleability. The acid catalysts can be used alone as one kind or can be used in combination of two or more kinds. Also, the amount of the acid catalyst used can be arbitrarily set according to, for example, the kind of the raw materials and the catalyst used and moreover the reaction conditions and is not particularly limited, but is preferably 0.01 to 100 parts by mass based on 100 parts by mass of the reaction raw materials.

Upon the reaction, a reaction solvent may be used. The reaction solvent is not particularly limited as long as the reaction of the aldehyde or the ketone used with the 2,2'-biphenol proceeds, and can be arbitrarily selected and used from publicly known solvents. Examples of the reaction solvent include water, methanol, ethanol, propanol, butanol, tetrahydrofuran, dioxane, ethylene glycol dimethyl ether, ethylene glycol diethyl ether, and a mixed solvent thereof. The solvents can be used alone as one kind or can be used in combination of two or more kinds.

Also, the amount of these reaction solvents used can be arbitrarily set according to, for example, the kind of the raw materials and the catalyst used and moreover the reaction conditions and is not particularly limited, but is preferably in the range of 0 to 2000 parts by mass based on 100 parts by mass of the reaction raw materials. Furthermore, the reaction temperature in the reaction can be arbitrarily selected according to the reactivity of the reaction raw materials and is not particularly limited, but is usually within the range of 10 to 200°C.

In order to obtain the polyphenol compound, a higher reaction temperature is more preferable. Specifically, the range of 60 to 200°C is preferable. The reaction method can be arbitrarily selected and used from publicly known approaches and is not particularly limited, and examples thereof include a method of charging the 2,2'-biphenol, the aldehyde or the ketone, and the catalyst in one portion, and a method of dropping the 2,2'-biphenol and the aldehyde or the ketone, in the presence of the catalyst. After the polycondensation reaction terminates, isolation of the obtained compound can be carried out according to a conventional method, and is not particularly limited. For example, by adopting a commonly used approach in which the temperature of the reaction vessel is elevated to 130 to 230°C in order to remove unreacted raw materials, catalyst, etc. present in the system, and volatile portions are removed at about 1 to 50 mmHg, the compound that is the target compound can be isolated.

Preferable examples of the reaction conditions include conditions involving using 1 mol to an excess of the 2,2'-biphenol and 0.001 to 1 mol of the acid catalyst based on 1 mol of the aldehyde or the ketone, and reacting them at 50 to 150°C at normal pressure for about 20 minutes to 100 hours.

The target compound can be isolated by a publicly known method after the reaction terminates. The compound represented by the above formula (0) which is the target compound can be obtained, for example, by concentrating the reaction solution, precipitating the reaction product by the addition of pure water, cooling the reaction solution to room temperature, then separating the precipitates by filtration, filtering and drying the obtained solid matter, then separating and purifying the solid matter from by-products by column chromatography, and distilling off the solvent, followed by filtration and drying.

### [Resin obtained with compound represented by formula (0) as monomer]

The compound represented by the above formula (0) can be used directly as a composition for use in film formation for lithography or optical component formation (hereinafter, also simply referred to as a "composition"). Also, a resin obtained with the compound represented by the above formula (0) as a monomer can be used as a composition. The resin is obtained, for example, by reacting the compound represented by the above formula (0) with a crosslinking compound.

Examples of the resin obtained with the compound represented by the above formula (0) as a monomer include resins having a structure represented by the following formula (2). That is, the composition of the present embodiment may contain a resin having a structure represented by the following formula (2).

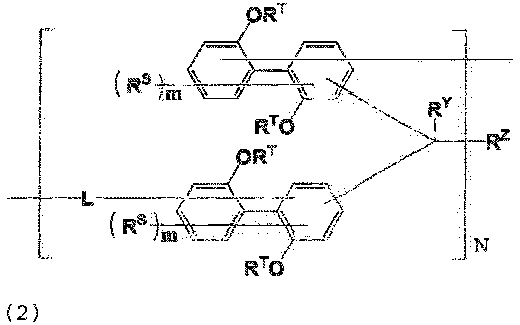

In the formula (2), L is an alkylene group, which has 1 to 30 carbon atoms, and which optionally has a substituent, an arylene group, which has 6 to 30 carbon atoms, and which optionally has a substituent, an alkoxylene group, which has 1 to 30 carbon atoms, and which optionally has a substituent or a single bond, wherein the alkylene group, the arylene group, and the alkoxylene group each optionally contain an ether bond, a ketone bond, or an ester bond. Each of the alkylene group and the alkoxylene group may be a linear, branched, or cyclic group.

R^{Y}, R^{Z}, R^{T}, R^{S}, N, and m are as defined in the description of the above formula (0).

The resin represented by the above formula (2) is preferably a resin having a structure represented by the following formula (3) from the viewpoint of the availability of raw materials. 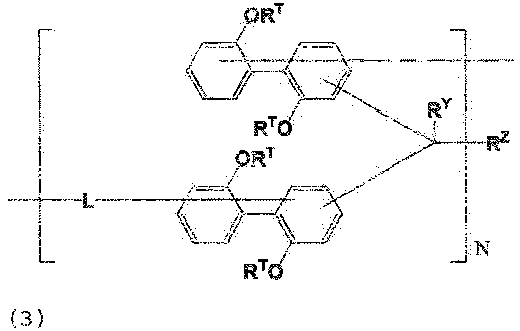 wherein R^{Y}, R^{Z}, R^{T}, N, and L are as defined above.

### [Method for producing resin obtained with compound represented by formula (0) as monomer]

The resin of the present embodiment is obtained by reacting the compound represented by the above formula (0) with a crosslinking compound. As the crosslinking compound, a publicly known monomer can be used without particular limitations as long as it can oligomerize or polymerize the compound represented by the above formula (0). Specific examples thereof include, but not particularly limited to, aldehydes, ketones, carboxylic acids, carboxylic acid halides, halogen-containing compounds, amino compounds, imino compounds, isocyanates, and unsaturated hydrocarbon group-containing compounds.

Specific examples of the resin having the structure represented by the above formula (2) include resins that are made novolac by, for example, a condensation reaction between the compound represented by the above formula (0) with an aldehyde and/or a ketone that is a crosslinking compound.

Herein, examples of the aldehyde used when making the compound represented by the above formula (0) novolac include, but not particularly limited to, formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarbaldehyde, phenanthrenecarbaldehyde, pyrenecarbaldehyde, and furfural. Examples of the ketone include the ketones listed above. Among them, formaldehyde is more preferable. These aldehydes and/or ketones can be used alone as one kind or may be used in combination of two or more kinds. The amount of the above aldehydes and/or ketones used is not particularly limited, but is preferably 0.2 to 5 mol and more preferably 0.5 to 2 mol based on 1 mol of the compound represented by the above formula (0).

An acid catalyst can also be used in the condensation reaction between the compound represented by the above formula (0) and the aldehyde and/or ketones. The acid catalyst used herein can be arbitrarily selected and used from publicly known catalysts and is not particularly limited. Inorganic acids and organic acids are widely known as such acid catalysts, and examples include, but not particularly limited to, inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, organic acids and solid acids are preferable from the viewpoint of production, and hydrochloric acid or sulfuric acid is preferable from the viewpoint of production such as easy availability and handleability. The acid catalysts can be used alone as one kind, or can be used in combination of two or more kinds.

Also, the amount of the acid catalyst used can be arbitrarily set according to, for example, the kind of the raw materials used and the catalyst and moreover the reaction conditions and is not particularly limited, but is preferably 0.01 to 100 parts by mass based on 100 parts by mass of the reaction raw materials. The aldehyde is not necessarily needed in the case of a copolymerization reaction with a compound having a nonconjugated double bond, such as indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, 5-vinylnorborn-2-ene, α-pinene, β-pinene, and limonene.

A reaction solvent can also be used in the condensation reaction between the compound represented by the above formula (0) and the aldehyde and/or ketones. The reaction solvent in the polycondensation can be arbitrarily selected and used from publicly known solvents and is not particularly limited, and examples include water, methanol, ethanol, propanol, butanol, tetrahydrofuran, dioxane, or a mixed solvent thereof. The solvents can be used alone as one kind, or can be used in combination of two or more kinds.

Also, the amount of these solvents used can be arbitrarily set according to, for example, the kind of the raw materials used and the catalyst and moreover the reaction conditions and is not particularly limited, but is preferably in the range of 0 to 2000 parts by mass based on 100 parts by mass of the reaction raw materials. Furthermore, the reaction temperature can be arbitrarily selected according to the reactivity of the reaction raw materials and is not particularly limited, but is usually within the range of 10 to 200°C. The reaction method can be arbitrarily selected and used from publicly known approaches and is not particularly limited, and there are a method of charging the compound represented by the above formula (0), the aldehyde and/or ketones, and the catalyst in one portion, and a method of dropping the compound represented by the above formula (0) and the aldehyde and/or ketones in the presence of the catalyst.

After the polycondensation reaction terminates, isolation of the obtained compound can be carried out according to a conventional method, and is not particularly limited. For example, by adopting a commonly used approach in which the temperature of the reaction vessel is elevated to 130 to 230°C in order to remove unreacted raw materials, catalyst, etc. present in the system, and volatile portions are removed at about 1 to 50 mmHg, a novolac resin that is the target compound can be isolated.

Herein, the resin having the structure represented by the above formula (2) may be a homopolymer of a compound represented by the above formula (0), or may be a copolymer with a further phenol. Herein, examples of the copolymerizable phenol include, but not particularly limited to, phenol, cresol, dimethylphenol, trimethylphenol, butylphenol, phenylphenol, diphenylphenol, naphthylphenol, resorcinol, methylresorcinol, catechol, butylcatechol, methoxyphenol, methoxyphenol, propylphenol, pyrogallol, and thymol.

The resin having the structure represented by the above formula (2) may be a copolymer with a polymerizable monomer other than the above-described further phenols. Examples of such a copolymerization monomer include, but not particularly limited to, naphthol, methylnaphthol, methoxynaphthol, dihydroxynaphthalene, indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, vinylnorbornene, pinene, and limonene. The resin having the structure represented by the above formula (3) may be a copolymer of two or more components (for example, a binary to quaternary system) composed of the compound represented by the above formula (0) and the above-described phenol, may be a copolymer of two or more components (for example, a binary to quaternary system) composed of the compound represented by the above formula (0) and the above-described copolymerization monomer, or may be a copolymer of three or more components (for example, a tertiary to quaternary system) composed of the compound represented by the above formula (0), the above-described phenol, and the above-described copolymerization monomer.

The molecular weight of the resin having the structure represented by the above formula (2) is not particularly limited, and the weight average molecular weight (Mw) in terms of polystyrene is preferably 500 to 30,000 and more preferably 750 to 20,000. The resin having the structure represented by the above formula (2) preferably has dispersibility (weight average molecular weight Mw/number average molecular weight Mn) within the range of 1.2 to 7 from the viewpoint of enhancing crosslinking efficiency while suppressing volatile components during baking. The above Mw and Mn can be determined by a method described in Examples mentioned later.

The resin having the structure represented by the above formula (2) preferably has high solubility in a solvent from the viewpoint of easier application to a wet process, etc. More specifically, in the case of using 1-methoxy-2-propanol (PGME) and/or propylene glycol monomethyl ether acetate (PGMEA) as a solvent, a solubility of 10% by mass or more in the solvent is preferable. Herein, the solubility in PGME and/or PGMEA is defined as "mass of the resin / (mass of the resin + mass of the solvent) × 100 (% by mass)". For example, when 10 g of the resin is dissolved in 90 g of PGMEA, the solubility of the resin in PGMEA is "10% by mass or more"; and when 10 g of the resin is not dissolved in 90 g of PGMEA, the solubility is "less than 10% by mass".

### [Method for purifying compound and/or resin]

The method for purifying the compound and/or the resin of the present embodiment comprises the steps of: obtaining a solution (S) by dissolving one or more selected from the compound represented by the above formula (0) and the resin obtained with the compound represented by the above formula (0) as a monomer in a solvent; and extracting impurities in the compound and/or the resin by bringing the obtained solution (S) into contact with an acidic aqueous solution (a first extraction step), wherein the solvent used in the step of obtaining the solution (S) contains a solvent that does not inadvertently mix with water.

In the first extraction step, the resin is preferably a resin obtained by a reaction between the compound represented by the above formula (1) and/or the compound represented by the formula (2) and a crosslinking compound. According to the purification method of the present embodiment, the contents of various metals that may be contained as impurities in the compound or the resin having a specific structure described above can be reduced.

More specifically, in the purification method of the present embodiment, the compound and/or the resin is dissolved in an organic solvent that does not inadvertently mix with water to obtain the solution (S), and further, extraction treatment can be carried out by bringing the solution (S) into contact with an acidic aqueous solution. Thereby, metals contained in the solution (S) are transferred to the aqueous phase, then the organic phase and the aqueous phase are separated, and thus the compound and/or the resin having a reduced metal content can be obtained.

The compound and/or the resin used in the purification method of the present embodiment may be alone, or may be a mixture of two or more kinds. Also, the compound and/or the resin may contain various surfactants, various crosslinking agents, various acid generating agents, various stabilizers, and the like.

The solvent that does not inadvertently mix with water used in the purification method of the present embodiment is not particularly limited, but is preferably an organic solvent that is safely applicable to semiconductor manufacturing processes, and specifically it is an organic solvent having a solubility in water at room temperature of less than 30%, more preferably less than 20%, and still more preferably less than 10%. The amount of the organic solvent.used is preferably 1 to 100 times the total mass of the compound and/or the resin to be used.

Specific examples of the solvent that does not inadvertently mix with water include, but not limited to, ethers such as diethyl ether and diisopropyl ether; esters such as ethyl acetate, n-butyl acetate, and isoamyl acetate; ketones such as methyl ethyl ketone, methyl isobutyl ketone, ethyl isobutyl ketone, cyclohexanone, cyclopentanone, 2-heptanone, and 2-pentanone; glycol ether acetates such as ethylene glycol monoethyl ether acetate, ethylene glycol monobutyl ether acetate, propylene glycol monomethyl ether acetate (PGMEA), and propylene glycol monoethyl ether acetate; aliphatic hydrocarbons such as n-hexane and n-heptane; aromatic hydrocarbons such as toluene and xylene; and halogenated hydrocarbons such as methylene chloride and chloroform. Among these, toluene, 2-heptanone, cyclohexanone, cyclopentanone, methyl isobutyl ketone, propylene glycol monomethyl ether acetate, ethyl acetate, and the like are preferable, methyl isobutyl ketone, ethyl acetate, cyclohexanone, and propylene glycol monomethyl ether acetate are more preferable, and methyl isobutyl ketone and ethyl acetate are still more preferable. Methyl isobutyl ketone, ethyl acetate, and the like have relatively high saturation solubility for the above compound and the resin comprising the compound as a constituent and a relatively low boiling point, and it is thus possible to reduce the load in the case of industrially distilling off the solvent and in the step of removing the solvent by drying. These solvents can be each used alone, and can be used as a mixture of two or more kinds.

The acidic aqueous solution used in the purification method of the present embodiment is arbitrarily selected from among aqueous solutions in which organic compounds or inorganic compounds are dissolved in water, generally known as acidic aqueous solutions. Examples of the acidic aqueous solution include, but not limited to, aqueous mineral acid solutions in which mineral acids such as hydrochloric acid, sulfuric acid, nitric acid, and phosphoric acid are dissolved in water; and aqueous organic acid solutions in which organic acids such as acetic acid, propionic acid, oxalic acid, malonic acid, succinic acid, fumaric acid, maleic acid, tartaric acid, citric acid, methanesulfonic acid, phenolsulfonic acid, p-toluenesulfonic acid, and trifluoroacetic acid are dissolved in water. These acidic aqueous solutions can be each used alone, and can be also used as a combination of two or more kinds. Among these acidic aqueous solutions, aqueous solutions of one or more mineral acids selected from the group consisting of hydrochloric acid, sulfuric acid, nitric acid, and phosphoric acid, or aqueous solutions of one or more organic acids selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid, and trifluoroacetic acid are preferable, aqueous solutions of sulfuric acid, nitric acid, and carboxylic acids such as acetic acid, oxalic acid, tartaric acid, and citric acid are more preferable, aqueous solutions of sulfuric acid, oxalic acid, tartaric acid, and citric acid are still more preferable, and an aqueous solution of oxalic acid is further preferable. It is considered that polyvalent carboxylic acids such as oxalic acid, tartaric acid, and citric acid coordinate with metal ions and provide a chelating effect, and thus tend to be capable of more effectively removing metals. As for water used herein, it is preferable to use water, the metal content of which is small, such as ion exchanged water, according to the purpose of the purification method of the present embodiment.

The pH of the acidic aqueous solution used in the purification method of the present embodiment is not particularly limited, but it is preferable to regulate the acidity of the aqueous solution in consideration of an influence on the compound or the resin. Normally, the pH of the acidic aqueous solution is about 0 to 5, and is preferably about pH 0 to 3.

The amount of the acidic aqueous solution used in the purification method of the present embodiment is not particularly limited, but it is preferable to regulate the amount from the viewpoint of reducing the number of extraction operations for removing metals and from the viewpoint of ensuring operability in consideration of the overall amount of fluid. From the above viewpoints, the amount of the acidic aqueous solution used is preferably 10 to 200 parts by mass, and more preferably 20 to 100 parts by mass, based on 100 parts by mass of the solution (S) .

In the purification method of the present embodiment, by bringing the acidic aqueous solution as described above into contact with the solution (S), metals can be extracted from the compound or the resin in the solution (S).

In the purification method of the present embodiment, it is preferable that the solution (S) further contains an organic solvent that inadvertently mixes with water. When the solution (S) contains an organic solvent that inadvertently mixes with water, there is a tendency that the amount of the compound and/or the resin charged can be increased, also the fluid separability is improved, and purification can be carried out at a high reaction vessel efficiency. The method for adding the organic solvent that inadvertently mixes with water is not particularly limited and may be, for example, any of a method involving adding it to the organic solvent-containing solution in advance, a method involving adding it to water or the acidic aqueous solution in advance, and a method involving adding it after bringing the organic solvent-containing solution into contact with water or the acidic aqueous solution. Among these, the method involving adding it to the organic solvent-containing solution in advance is preferable from the viewpoint of the workability of operations and the ease of managing the amount.

The organic solvent that inadvertently mixes with water used in the purification method of the present embodiment is not particularly limited, but is preferably an organic solvent that is safely applicable to semiconductor manufacturing processes. The amount of the organic solvent used that inadvertently mixes with water is not particularly limited as long as the solution phase and the aqueous phase separate, but is preferably 0.1 to 100 times, more preferably 0.1 to 50 times, and further preferably 0.1 to 20 times the total mass of the compound and the resin to be used.

Specific examples of the organic solvent used in the purification method of the present embodiment that inadvertently mixes with water include, but not limited to, ethers such as tetrahydrofuran and 1,3-dioxolane; alcohols such as methanol, ethanol, and isopropanol; ketones such as acetone and N-methylpyrrolidone; aliphatic hydrocarbons such as glycol ethers such as ethylene glycol monoethyl ether, ethylene glycol monobutyl ether, propylene glycol monomethyl ether (PGME), and propylene glycol monoethyl ether. Among these, N-methylpyrrolidone, propylene glycol monomethyl ether, and the like are preferable, and N-methylpyrrolidone and propylene glycol monomethyl ether are more preferable. These solvents can be each used alone, and can be used as a mixture of two or more kinds.

The temperature when extraction treatment is carried out is generally in the range of 20 to 90°C, and preferably 30 to 80°C. The extraction operation is carried out, for example, by thoroughly mixing the solution (S) and the acidic aqueous solution by stirring or the like and then leaving the obtained mixed solution to stand still. Thereby, metals contained in the solution (S) are transferred to the aqueous phase. Also, by this operation, the acidity of the solution is lowered, and the degradation of the compound and/or the resin can be suppressed.

By being left to stand still, the mixed solution is separated into an aqueous phase and a solution phase containing the compound and/or the resin and the solvents, and thus the solution phase is recovered by decantation. The time for leaving the mixed solution to stand still is not particularly limited, but it is preferable to regulate the time for leaving the mixed solution to stand still from the viewpoint of attaining good separation of the solution phase containing the solvents and the aqueous phase. Normally, the time for leaving the mixed solution to stand still is 1 minute or longer, preferably 10 minutes or longer, and more preferably 30 minutes or longer. While the extraction treatment may be carried out once, it is effective to repeat mixing, leaving-to-stand-still, and separating operations multiple times.

It is preferable that the purification method of the present embodiment includes the step of extracting impurities in the compound or the resin by further bringing the solution phase containing the compound or the resin into contact with water after the first extraction step (the second extraction step). Specifically, for example, it is preferable that after the above extraction treatment is carried out using an acidic aqueous solution, the solution phase that is extracted and recovered from the aqueous solution and that contains the compound and/or the resin and the solvents is further subjected to extraction treatment with water. The extraction treatment with water is not particularly limited, and can be carried out, for example, by thoroughly mixing the solution phase and water by stirring or the like and then leaving the obtained mixed solution to stand still. The mixed solution after being left to stand still is separated into an aqueous phase and a solution phase containing the compound and/or the resin and the solvents, and thus the solution phase can be recovered by decantation.

Water used herein is preferably water, the metal content of which is small, such as ion exchanged water, according to the purpose of the present embodiment. While the extraction treatment may be carried out once, it is effective to repeat mixing, leaving-to-stand-still, and separating operations multiple times. The proportions of both used in the extraction treatment and temperature, time, and other conditions are not particularly limited, and may be the same as those of the previous contact treatment with the acidic aqueous solution.

Water that is possibly present in the thus-obtained solution containing the compound and/or the resin can be easily removed by performing vacuum distillation or a like operation. Also, if required, the concentration of the compound and/or the resin can be regulated to be any concentration by adding a solvent to the solution.

The method for isolating the compound and/or the resin from the obtained solution containing the compound and/or the resin and the solvents is not particularly limited, and publicly known methods can be carried out, such as reduced-pressure removal, separation by reprecipitation, and a combination thereof. Publicly known treatments such as concentration operation, filtration operation, centrifugation operation, and drying operation can be carried out if required.

### [Composition]

The composition of the present embodiment contains one or more selected from the group consisting of the compound represented by the above formula (0) and the resin obtained with the compound represented by the above formula (0) as a monomer.

The composition of the present embodiment can be a film forming composition for lithography or an optical component forming composition.

### [Film forming composition for lithography for chemical amplification type resist purpose]

The film forming composition for lithography for chemical amplification type resist purposes (hereinafter, also referred to as a "resist composition") of the present embodiment contains one or more resist base materials selected from the group consisting of the compound represented by the above formula (0) and the resin obtained with the compound represented by the above formula (0) as a monomer.

It is preferable that the composition (resist composition) of the present embodiment should further contain a solvent. Examples of the solvent can include, but not particularly limited to, ethylene glycol monoalkyl ether acetates such as ethylene glycol monomethyl ether acetate, ethylene glycol monoethyl ether acetate, ethylene glycol mono-n-propyl ether acetate, and ethylene glycol mono-n-butyl ether acetate; ethylene glycol monoalkyl ethers such as ethylene glycol monomethyl ether and ethylene glycol monoethyl ether; propylene glycol monoalkyl ether acetates such as propylene glycol monomethyl ether acetate (PGMEA), propylene glycol monoethyl ether acetate, propylene glycol mono-n-propyl ether acetate, and propylene glycol mono-n-butyl ether acetate; propylene glycol monoalkyl ethers such as propylene glycol monomethyl ether (PGME) and propylene glycol monoethyl ether; ester lactates such as methyl lactate, ethyl lactate, n-propyl lactate, n-butyl lactate, and n-amyl lactate; aliphatic carboxylic acid esters such as methyl acetate, ethyl acetate, n-propyl acetate, n-butyl acetate, n-amyl acetate, n-hexyl acetate, methyl propionate, and ethyl propionate; other esters such as methyl 3-methoxypropionate, ethyl 3-methoxypropionate, methyl 3-ethoxypropionate, ethyl 3-ethoxypropionate, methyl 3-methoxy-2-methylpropionate, 3-methoxybutylacetate, 3-methyl-3-methoxybutylacetate, butyl 3-methoxy-3-methylpropionate, butyl 3-methoxy-3-methylbutyrate, methyl acetoacetate, methyl pyruvate, and ethyl pyruvate; aromatic hydrocarbons such as toluene and xylene; ketones such as 2-heptanone, 3-heptanone, 4-heptanone, cyclopentanone (CPN), and cyclohexanone (CHN); amides such as N,N-dimethylformamide, N-methylacetamide, N,N-dimethylacetamide, and N-methylpyrrolidone; and lactones such as γ-lactone. These solvents may be used alone or in combination of two or more kinds.

The solvent used in the present embodiment is preferably a safe solvent, more preferably at least one selected from PGMEA, PGME, CHN, CPN, 2-heptanone, anisole, butyl acetate, ethyl propionate, and ethyl lactate, and still more preferably at least one selected from PGMEA, PGME, and CHN.

In the present embodiment, the amount of the solid component and the amount of the solvent are not particularly limited, but preferably the solid component is 1 to 80% by mass and the solvent is 20 to 99% by mass, more preferably the solid component is 1 to 50% by mass and the solvent is 50 to 99% by mass, still more preferably the solid component is 2 to 40% by mass and the solvent is 60 to 98% by mass, and particularly preferably the solid component is 2 to 10% by mass and the solvent is 90 to 98% by mass, based on 100% by mass of the total mass of the amount of the solid component and the solvent.

The composition (resist composition) of the present embodiment may further contain at least one selected from the group consisting of an acid generating agent (C), an acid crosslinking agent (G), an acid diffusion controlling agent (E), and a further component (F), as other solid components. In the present specification, the "solid components" refer to components except for the solvent.

Herein, as the acid generating agent (C), the acid crosslinking agent (G), the acid diffusion controlling agent (E), and the further component (F), publicly known agents can be used, and they are not particularly limited, but those described in International Publication No. WO 2013/024778 are preferable.

### [Content ratio of each component]

In the resist composition of the present embodiment, the content of the compound and/or the resin used as a resist base material is not particularly limited, but is preferably 50 to 99.4% by mass of the total mass of the solid components (summation of solid components including the resist base material, and optionally used components such as acid generating agent (C), acid crosslinking agent (G), acid diffusion controlling agent (E), and further component (F) (also referred to as "optional component (F)"), hereinafter the same), more preferably 55 to 90% by mass, still more preferably 60 to 80% by mass, and particularly preferably 60 to 70% by mass. When the content of the compound and/or the resin used as a resist base material falls within the above range, there is a tendency that resolution is further improved, and line edge roughness (LER) is further decreased.

When both of the compound and the resin are contained as a resist base material, the above content refers to the total amount of these components.

### [Further component (F)]

To the resist composition of the present embodiment, if required, as a component other than the resist base material, the acid generating agent (C), the acid crosslinking agent (G) and the acid diffusion controlling agent (E), one kind or two kinds or more of various additive agents such as a dissolution promoting agent, dissolution controlling agent, sensitizing agent, surfactant, organic carboxylic acid or oxo acid of phosphor or derivative thereof, thermal and/or light curing catalyst, polymerization inhibitor, flame retardant, filler, coupling agent, thermosetting resin, light curable resin, dye, pigment, thickener, lubricant, antifoaming agent, leveling agent, ultraviolet absorber, surfactant, colorant, and nonionic surfactant can be added within the range not inhibiting the objects of the present invention. In the present specification, the further component (F) is also referred to as an optional component (F).

In the resist composition of the present embodiment, the contents of the resist base material (hereinafter, also referred to as a "component (A)"), the acid generating agent (C), the acid crosslinking agent (G), the acid diffusion controlling agent (E), and the optional component (F) (the component (A)/the acid generating agent (C)/the acid crosslinking agent (G)/the acid diffusion controlling agent (E)/the optional component (F)) are preferably 50 to 99.4/0.001 to 49/0.5 to 49/0.001 to 49/0 to 49, more preferably 55 to 90/1 to 40/0.5 to 40/0.01 to 10/0 to 5, further preferably 60 to 80/3 to 30/1 to 30/0.01 to 5/0 to 1, and particularly preferably 60 to 70/10 to 25/2 to 20/0.01 to 3/0% by mass based on solid matter.

The content ratio of each component is selected from each range so that the summation thereof is 100% by mass. When the content ratio of each component falls within the above range, performance such as sensitivity, resolution, and developability tends to be excellent.

The resist composition of the present embodiment is generally prepared by dissolving each component in a solvent upon use into a homogeneous solution, and then if required, filtering through a filter or the like with a pore diameter of about 0.2 µm, for example.

The resist composition of the present embodiment can contain an additional resin other than the resin of the present embodiment, within the range not inhibiting the objects of the present invention. Examples of the further resin include, but not particularly limited to, a novolac resin, polyvinyl phenols, polyacrylic acid, polyvinyl alcohol, a styrene-maleic anhydride resin, and polymers containing an acrylic acid, vinyl alcohol or vinylphenol as a monomeric unit, and derivatives thereof. The content of the further resin is not particularly limited and is arbitrarily adjusted according to the kind of the component (A) to be used, and is preferably 30 parts by mass or less per 100 parts by mass of the component (A), more preferably 10 parts by mass or less, still more preferably 5 parts by mass or less, and particularly preferably 0 part by mass.

### [Physical properties and the like of resist composition]

The resist composition of the present embodiment can be used to form an amorphous film by spin coating. Also, the resist composition of the present embodiment can be applied to a general semiconductor production process. Any of positive type and negative type resist patterns can be individually prepared depending on the type of the compound represented by the above formula (0) or the resin obtained with this compound as a monomer and/or the kind of a developing solution to be used.

In the case of a positive type resist pattern, the dissolution rate of the amorphous film formed by spin coating with the resist composition of the present embodiment in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, there is a tendency that the above portion is insoluble in a developing solution, and thus the amorphous film easily forms a resist. When the dissolution rate is 0.0005 angstrom/sec or more, the resolution may improve. It is presumed that this is because due to the change in the solubility before and after exposure of the compound represented by the above formula (0) and/or the resin comprising the compound as a constituent, contrast at the interface between the exposed portion being dissolved in a developing solution and the unexposed portion not being dissolved in a developing solution is increased. Also, effects of reducing LER and defects are seen.

In the case of a negative type resist pattern, the dissolution rate of the amorphous film formed by spin coating with the resist composition of the present embodiment in a developing solution at 23°C is preferably 10 angstrom/sec or more. When the dissolution rate is 10 angstrom/sec or more, the amorphous film more easily dissolves in a developing solution, and is suitable for a resist. When the dissolution rate is 10 angstrom/sec or more, the resolution may improve. It is presumed that this is because the micro surface portion of the compound represented by the above formula (0) and/or the resin comprising the compound as a constituent dissolves, and LER is reduced. Also, effects of reducing defects are seen.

The dissolution rate can be determined by immersing the amorphous film in a developing solution for a predetermined period of time at 23°C and then measuring the film thickness before and after immersion by a publicly known method such as visual, ellipsometric, or QCM method.

In the case of a positive type resist pattern, the dissolution rate of the portion exposed by radiation such as KrF excimer laser, extreme ultraviolet, electron beam or X-ray, of the amorphous film formed by spin coating with the resist composition of the present embodiment, in a developing solution at 23°C is preferably 10 angstrom/sec or more. When the dissolution rate is 10 angstrom/sec or more, the amorphous film more easily dissolves in a developing solution, and is suitable for a resist. When the dissolution rate is 10 angstrom/sec or more, the resolution may improve. It is presumed that this is because the micro surface portion of the compound represented by the above formula (0) and/or the resin comprising the compound as a constituent dissolves, and LER is reduced. Also, effects of reducing defects are seen.

In the case of a negative type resist pattern, the dissolution rate of the portion exposed by radiation such as KrF excimer laser, extreme ultraviolet, electron beam or X-ray, of the amorphous film formed by spin coating with the resist composition of the present embodiment, in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, there is a tendency that the above portion is insoluble in a developing solution, and thus the amorphous film easily forms a resist. When the dissolution rate is 0.0005 angstrom/sec or more, the resolution may improve. It is presumed that this is because due to the change in the solubility before and after exposure of the compound represented by the above formula (0) and/or the resin comprising the compound as a constituent, contrast at the interface between the unexposed portion being dissolved in a developing solution and the exposed portion not being dissolved in a developing solution is increased. Also, effects of reducing LER and defects are seen.

### [Film forming composition for lithography for non-chemical amplification type resist purpose]

The component (A) to be contained in the film forming composition for lithography for non-chemical amplification type resist purposes (hereinafter, also referred to as a "radiation-sensitive composition") of the present embodiment is used in combination with the optically active diazonaphthoquinone compound (B) mentioned later and is useful as a base material for positive type resists that becomes a compound easily soluble in a developing solution by irradiation with g-ray, h-ray, i-ray, KrF excimer laser, ArF excimer laser, extreme ultraviolet, electron beam, or X-ray. Although the properties of the component (A) are not largely altered by g-ray, h-ray, i-ray, KrF excimer laser, ArF excimer laser, extreme ultraviolet, electron beam, or X-ray, the optically active diazonaphthoquinone compound (B) poorly soluble in a developing solution is converted to an easily soluble compound, and a resist pattern can therefore be formed in a development step.

Since the component (A) to be contained in the radiation-sensitive composition of the present embodiment is a relatively low molecular weight compound, the obtained resist pattern has very small roughness. Also, in the above formula (0), at least one selected from the group consisting of R^{S} is preferably a group containing an iodine atom. In the case of applying the component (A) having such a group containing an iodine atom to the radiation-sensitive composition of the present embodiment, the ability to absorb radiation such as electron beam, extreme ultraviolet (EUV), or X-ray is increased. As a result, this enables the enhancement of the sensitivity, which is preferable.

The glass transition temperature of the component (A) to be contained in the radiation-sensitive composition of the present embodiment is preferably 100°C or higher, more preferably 120°C or higher, still more preferably 140°C or higher, and particularly preferably 150°C or higher. The upper limit of the glass transition temperature of the component (A) is not particularly limited and is, for example, 400°C. When the glass transition temperature of the component (A) falls within the above range, the resulting radiation-sensitive composition tends to have heat resistance capable of maintaining a pattern shape in a semiconductor lithography process, and improve performance such as high resolution.

The heat of crystallization determined by the differential scanning calorimetry of the glass transition temperature of the component (A) to be contained in the radiation-sensitive composition of the present embodiment is preferably less than 20 J/g. (Crystallization temperature) - (Glass transition temperature) is preferably 70°C or more, more preferably 80°C or more, still more preferably 100°C or more, and particularly preferably 130°C or more. When the heat of crystallization is less than 20 J/g or (Crystallization temperature) - (Glass transition temperature) falls within the above range, there is a tendency that the radiation-sensitive composition easily forms an amorphous film by spin coating, can maintain film formability necessary for a resist over a long period, and can improve resolution.

In the present embodiment, the above heat of crystallization, crystallization temperature, and glass transition temperature can be determined by differential scanning calorimetry using "DSC/TA-50WS" manufactured by Shimadzu Corp. For example, about 10 mg of a sample is placed in an unsealed container made of aluminum, and the temperature is raised to the melting point or more at a temperature increase rate of 20°C/min in a nitrogen gas stream (50 mL/min). After quenching, again the temperature is raised to the melting point or more at a temperature increase rate of 20°C/min in a nitrogen gas stream (30 mL/min). After further quenching, again the temperature is raised to 400°C at a temperature increase rate of 20°C/min in a nitrogen gas stream (30 mL/min) . The temperature at the middle point (where the specific heat is changed into the half) of steps in the baseline shifted in a step-like pattern is defined as the glass transition temperature (Tg). The temperature of the subsequently appearing exothermic peak is defined as the crystallization temperature. The heat is determined from the area of a region surrounded by the exothermic peak and the baseline and defined as the heat of crystallization.

The component (A) to be contained in the radiation-sensitive composition of the present embodiment is preferably low sublimable at 100 or lower, preferably 120°C or lower, more preferably 130°C or lower, still more preferably 140°C or lower, and particularly preferably 150°C or lower at normal pressure. The low sublimability means that in thermogravimetry, weight reduction when the resist base material is kept at a predetermined temperature for 10 minutes is 10% or less, preferably 5% or less, more preferably 3% or less, still more preferably 1% or less, and particularly preferably 0.1% or less. The low sublimability can prevent an exposure apparatus from being contaminated by outgassing upon exposure. In addition, a good pattern shape with low roughness can be obtained.

The component (A) to be contained in the radiation-sensitive composition of the present embodiment dissolves at preferably 1% by mass or more, more preferably 5% by mass or more, and still more preferably 10% by mass or more at 23°C in a solvent that is selected from the group consisting of propylene glycol monomethyl ether acetate (PGMEA), propylene glycol monomethyl ether (PGME), cyclohexanone (CHN), cyclopentanone (CPN), 2-heptanone, anisole, butyl acetate, ethyl propionate, and ethyl lactate and exhibits the highest ability to dissolve the component (A). Particularly preferably, the component (A) dissolves at 20% by mass or more at 23°C in a solvent that is selected from the group consisting of PGMEA, PGME, and CHN and exhibits the highest ability to dissolve the resist base material (A). Particularly preferably, the component (A) dissolves at 20% by mass or more at 23°C in PGMEA. When the above conditions are met, the radiation-sensitive composition is easily used in a semiconductor production process at a full production scale.

### [Optically active diazonaphthoquinone compound (B)]

The optically active diazonaphthoquinone compound (B) to be contained in the radiation-sensitive composition of the present embodiment is a diazonaphthoquinone substance including a polymer or non-polymer optically active diazonaphthoquinone compound and is not particularly limited as long as it is generally used as a photosensitive component (sensitizing agent) in positive type resist compositions. One kind or two or more kinds can be optionally selected and used.

The component (B) is preferably a compound obtained by reacting naphthoquinonediazide sulfonic acid chloride, benzoquinonediazide sulfonic acid chloride, or the like with a low molecular weight compound or a high molecular weight compound having a functional group condensable with these acid chlorides. Herein, examples of the above functional group condensable with the acid chlorides include, but not particularly limited to, a hydroxyl group and an amino group. Particularly, a hydroxyl group is preferable. Examples of the compound containing a hydroxyl group condensable with the acid chlorides can include, but not particularly limited to, hydroquinone; resorcin; hydroxybenzophenones such as 2,4-dihydroxybenzophenone, 2,3,4-trihydroxybenzophenone, 2,4,6-trihydroxybenzophenone, 2,4,4'-trihydroxybenzophenone, 2,3,4,4'-tetrahydroxybenzophenone, 2,2',4,4'-tetrahydroxybenzophenone, and 2,2',3,4,6'-pentahydroxybenzophenone; hydroxyphenylalkanes such as bis(2,4-dihydroxyphenyl)methane, bis(2,3,4-trihydroxyphenyl)methane, and bis(2,4-dihydroxyphenyl)propane; and hydroxytriphenylmethanes such as 4,4',3",4"-tetrahydroxy-3,5,3',5'-tetramethyltriphenylmethane and 4,4',2",3",4"-pentahydroxy-3,5,3',5'-tetramethyltriphenylmethane.

Preferable examples of the acid chloride such as naphthoquinonediazide sulfonic acid chloride or benzoquinonediazide sulfonic acid chloride include 1,2-naphthoquinonediazide-5-sulfonyl chloride and 1,2-naphthoquinonediazide-4-sulfonyl chloride.

The radiation-sensitive composition of the present embodiment is preferably prepared by, for example, dissolving each component in a solvent upon use into a homogeneous solution, and then if required, filtering through a filter or the like with a pore diameter of about 0.2 µm, for example.

### [Properties of radiation-sensitive composition]

The radiation-sensitive composition of the present embodiment can be used to form an amorphous film by spin coating. Also, the radiation-sensitive composition of the present embodiment can be applied to a general semiconductor production process. Any of positive type and negative type resist patterns can be individually prepared depending on the kind of a developing solution to be used.

In the case of a positive type resist pattern, the dissolution rate of the amorphous film formed by spin coating with the radiation-sensitive composition of the present embodiment in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, there is a tendency that the above portion is insoluble in a developing solution, and thus the amorphous film easily forms a resist. When the dissolution rate is 0.0005 angstrom/sec or more, the resolution may improve. It is presumed that this is because due to the change in the solubility before and after exposure of the compound represented by the above formula (1) and/or (2) and/or the resin comprising the compound as a constituent, contrast at the interface between the exposed portion being dissolved in a developing solution and the unexposed portion not being dissolved in a developing solution is increased. Also, effects of reducing LER and defects are seen.

In the case of a negative type resist pattern, the dissolution rate of the amorphous film formed by spin coating with the radiation-sensitive composition of the present embodiment in a developing solution at 23°C is preferably 10 angstrom/sec or more. When the dissolution rate is 10 angstrom/sec or more, the amorphous film more easily dissolves in a developing solution, and is suitable for a resist. When the dissolution rate is 10 angstrom/sec or more, the resolution may improve. It is presumed that this is because the micro surface portion of the compound represented by the above formula (1) and/or (2) and/or the resin comprising the compound as a constituent dissolves, and LER is reduced. Also, effects of reducing defects are seen.

The dissolution rate can be determined by immersing the amorphous film in a developing solution for a predetermined period of time at 23°C and then measuring the film thickness before and after immersion by a publicly known method such as visual, ellipsometric, or QCM method.

In the case of a positive type resist pattern, the dissolution rate of the exposed portion after irradiation with radiation such as KrF excimer laser, extreme ultraviolet, electron beam or X-ray, or after heating at 20 to 500°C, of the amorphous film formed by spin coating with the radiation-sensitive composition of the present embodiment, in a developing solution at 23°C is preferably 10 angstrom/sec or more, more preferably 10 to 10000 angstrom/sec, and still more preferably 100 to 1000 angstrom/sec. When the dissolution rate is 10 angstrom/sec or more, the amorphous film more easily dissolves in a developing solution, and is suitable for a resist. When the dissolution rate is 10000 angstrom/sec or less, the resolution may improve. It is presumed that this is because the micro surface portion of the compound represented by the above formula (0) and/or the resin comprising the compound as a constituent dissolves, and LER is reduced. Also, effects of reducing defects are seen.

In the case of a negative type resist pattern, the dissolution rate of the exposed portion after irradiation with radiation such as KrF excimer laser, extreme ultraviolet, electron beam or X-ray, or after heating at 20 to 500°C, of the amorphous film formed by spin coating with the radiation-sensitive composition of the present embodiment, in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, there is a tendency that the above portion is insoluble in a developing solution, and thus the amorphous film easily forms a resist. When the dissolution rate is 0.0005 angstrom/sec or more, the resolution may improve. It is presumed that this is because due to the change in the solubility before and after exposure of the compound represented by the above formula (0) and/or the resin comprising the compound as a constituent, contrast at the interface between the unexposed portion being dissolved in a developing solution and the exposed portion not being dissolved in a developing solution is increased. Also, effects of reducing LER and defects are seen.

### [Content ratio of each component]

In the radiation-sensitive composition of the present embodiment, the content of the component (A) is preferably 1 to 99% by mass of the total weight of the solid components (summation of the component (A), the optically active diazonaphthoquinone compound (B), and optionally used solid components such as further component (D), hereinafter the same), more preferably 5 to 95% by mass, still more preferably 10 to 90% by mass, and particularly preferably 25 to 75% by mass. When the content of the component (A) falls within the above range, there is a tendency that the radiation-sensitive composition of the present embodiment can produce a pattern with high sensitivity and low roughness.

In the radiation-sensitive composition of the present embodiment, the content of the optically active diazonaphthoquinone compound (B) is preferably 1 to 99% by mass of the total weight of the solid components (summation of the component (A), the optically active diazonaphthoquinone compound (B), and optionally used solid components such as further component (D), hereinafter the same), more preferably 5 to 95% by mass, still more preferably 10 to 90% by mass, and particularly preferably 25 to 75% by mass. When the content of the optically active diazonaphthoquinone compound (B) falls within the above range, there is a tendency that the radiation-sensitive composition of the present embodiment can produce a pattern with high sensitivity and low roughness.

### [Further component (D)]

To the radiation-sensitive composition of the present embodiment, if required, as a component other than the component (A) and the optically active diazonaphthoquinone compound (B), one kind or two kinds or more of various additive agents such as an acid generating agent, acid crosslinking agent, acid diffusion controlling agent, dissolution promoting agent, dissolution controlling agent, sensitizing agent, surfactant, organic carboxylic acid or oxo acid of phosphor or derivative thereof, thermal and/or light curing catalyst, polymerization inhibitor, flame retardant, filler, coupling agent, thermosetting resin, light curable resin, dye, pigment, thickener, lubricant, antifoaming agent, leveling agent, ultraviolet absorber, surfactant, colorant, and nonionic surfactant can be added within the range not inhibiting the objects of the present invention. In the present specification, the further component (D) is also referred to as an optional component (D).

In the radiation-sensitive composition of the present embodiment, the content ratio of each component (the component (A)/the optically active diazonaphthoquinone compound (B)/the optional component (D)) is
preferably 1 to 99/99 to 1/0 to 98,
more preferably 5 to 95/95 to 5/0 to 49,
further preferably 10 to 90/90 to 10/0 to 10,
further more preferably 20 to 80/80 to 20/0 to 5, and particularly preferably 25 to 75/75 to 25/0% by mass based on the solid components.

The content ratio of each component is selected from each range so that the summation thereof is 100% by mass. When the content ratio of each component falls within the above range, the radiation-sensitive composition of the present embodiment tends to be excellent in performance such as sensitivity and resolution, in addition to roughness.

The radiation-sensitive composition of the present embodiment may contain a further resin within the range not inhibiting the objects of the present invention. Examples of such a further resin include a novolac resin, polyvinyl phenols, polyacrylic acid, polyvinyl alcohol, a styrene-maleic anhydride resin, and polymers containing an acrylic acid, vinyl alcohol or vinylphenol as a monomeric unit, and derivatives thereof. The content of these resins, which is arbitrarily adjusted according to the kind of the component (A) to be used, is preferably 30 parts by mass or less per 100 parts by mass of the component (A), more preferably 10 parts by mass or less; still more preferably 5 parts by mass or less, and particularly preferably 0 part by mass.

### [Resist pattern formation method]

The resist pattern formation method of the present embodiment includes the steps of: forming a photoresist layer on a substrate using the above resist composition or radiation-sensitive composition of the present embodiment; and then irradiating a predetermined region of the photoresist layer with radiation for development. More specifically, the resist pattern formation method of the present embodiment includes the steps of: forming a resist film on a substrate using the above resist composition or radiation-sensitive composition of the present embodiment; exposing the formed resist film; and developing the resist film, thereby forming a resist pattern. The resist pattern according to the present embodiment can also be formed as an upper layer resist in a multilayer process.

Examples of the resist pattern formation method include, but not particularly limited to, the following methods. A resist film is formed by coating a conventionally publicly known substrate with the above resist composition or radiation-sensitive composition using a coating means such as spin coating, flow casting coating, and roll coating. Examples of the conventionally publicly known substrate include, but not particularly limited to, a substrate for electronic components, and the one having a predetermined wiring pattern formed thereon, or the like. More specific examples include a substrate made of a metal such as a silicon wafer, copper, chromium, iron and aluminum, and a glass substrate. Examples of a wiring pattern material include copper, aluminum, nickel, and gold. Also if required, the substrate may be a substrate having an inorganic and/or organic film provided thereon. Examples of the inorganic film include an inorganic antireflection film (inorganic BARC). Examples of the organic film include an organic antireflection film (organic BARC). The substrate may be subjected to surface treatment with hexamethylene disilazane or the like.

Next, the substrate coated with the resist composition or the radiation-sensitive composition is heated if required. The heating conditions vary according to the compounding composition of the resist composition or the radiation-sensitive composition, or the like, but are preferably 20 to 250°C, and more preferably 20 to 150°C. By heating, the adhesiveness of a resist to a substrate tends to improve, which is preferable. Then, the resist film is exposed to a desired pattern by any radiation selected from the group consisting of visible light, ultraviolet, excimer laser, electron beam, extreme ultraviolet (EUV), X-ray, and ion beam. The exposure conditions or the like are arbitrarily selected according to the compounding composition of the resist composition or radiation-sensitive composition, or the like. In the present embodiment, in order to stably form a fine pattern with a high degree of accuracy in exposure, the resist film is preferably heated after radiation irradiation. The heating conditions vary according to the compounding composition of the resist composition or radiation-sensitive composition, or the like, but are preferably 20 to 250°C, and more preferably 20 to 150°C.

Next, by developing the exposed resist film in a developing solution, a predetermined resist pattern is formed. As a developing solution, a solvent having a solubility parameter (SP value) close to that of the compound represented by the formula (1) or (2) or the resin obtained with the compound represented by the formula (1) or (2) as a monomer to be used is preferably selected. A polar solvent such as a ketone-based solvent, an ester-based solvent, an alcohol-based solvent, an amide-based solvent, and an ether-based solvent; and a hydrocarbon-based solvent, or an alkaline aqueous solution can be used.

Examples of the ketone-based solvent include 1-octanone, 2-octanone, 1-nonanone, 2-nonanone, acetone, 4-heptanone, 1-hexanone, 2-hexanone, diisobutyl ketone, cyclohexanone, methylcyclohexanone, phenylacetone, methyl ethyl ketone, methyl isobutyl ketone, acetylacetone, acetonylacetone, ionone, diacetonyl alcohol, acetyl carbinol, acetophenone, methyl naphthyl ketone, isophorone, and propylene carbonate.

Examples of the ester-based solvent include methyl acetate, butyl acetate, ethyl acetate, isopropyl acetate, amyl acetate, propylene glycol monomethyl ether acetate, ethylene glycol monoethyl ether acetate, diethylene glycol monobutyl ether acetate, diethylene glycol monoethyl ether acetate, ethyl-3-ethoxypropionate, 3-methoxybutyl acetate, 3-methyl-3-methoxybutyl acetate, methyl formate, ethyl formate, butyl formate, propyl formate, ethyl lactate, butyl lactate, and propyl lactate.

Examples of the alcohol-based solvent include an alcohol such as methyl alcohol, ethyl alcohol, n-propyl alcohol, isopropyl alcohol (2-propanol), n-butyl alcohol, sec-butyl alcohol, tert-butyl alcohol, isobutyl alcohol, n-hexyl alcohol, 4-methyl-2-pentanol, n-heptyl alcohol, n-octyl alcohol, and n-decanol; a glycol-based solvent such as ethylene glycol, diethylene glycol, and triethylene glycol; and a glycol ether-based solvent such as ethylene glycol monomethyl ether, propylene glycol monomethyl ether, ethylene glycol monoethyl ether, propylene glycol monoethyl ether, diethylene glycol monomethyl ether, triethylene glycol monoethyl ether, and methoxymethyl butanol.

Examples of the ether-based solvent include dioxane and tetrahydrofuran in addition to the glycol ether-based solvents.

Examples of the amide-based solvent include N-methyl-2-pyrrolidone, N,N-dimethylacetamide, N,N-dimethylformamide, phosphoric hexamethyltriamide, and 1,3-dimethyl-2-imidazolidinone.

Examples of the hydrocarbon-based solvent include an aromatic hydrocarbon-based solvent such as toluene and xylene; and an aliphatic hydrocarbon-based solvent such as pentane, hexane, octane, and decane.

A plurality of above solvents may be mixed, or the solvent may be used by mixing the solvent with a solvent other than those described above or water within the range having performance. From the viewpoint of sufficiently exhibiting the effect of the present invention, the water content ratio as the whole developing solution is preferably less than 70% by mass, more preferably less than 50% by mass, still more preferably less than 30% by mass, and further preferably less than 10% by mass. Particularly preferably, the developing solution is substantially moisture free. That is, the content of the organic solvent in the developing solution is preferably 30% by mass or more and 100% by mass or less based on the total amount of the developing solution, more preferably 50% by mass or more and 100% by mass or less, still more preferably 70% by mass or more and 100% by mass or less, further preferably 90% by mass or more and 100% by mass or less, and particularly preferably 95% by mass or more and 100% by mass or less.

Examples of the alkaline aqueous solution include an alkaline compound such as mono-, di- or trialkylamines, mono-, di- or tri-alkanolamines, heterocyclic amines, tetramethyl ammonium hydroxide (TMAH), and choline.

Particularly, the developing solution is preferably a developing solution containing at least one kind of solvent selected from a ketone-based solvent, an ester-based solvent, an alcohol-based solvent, an amide-based solvent, and an ether-based solvent from the viewpoint of improving resist performance such as resolution and roughness of the resist pattern.

The vapor pressure of the developing solution is preferably 5 kPa or less at 20°C, more preferably 3 kPa or less, and still more preferably 2 kPa or less. When the vapor pressure of the developing solution is 5 kPa or less, there is a tendency that the evaporation of the developing solution on the substrate or in a developing cup is inhibited to improve temperature uniformity within a wafer surface, thereby resulting in improvement in size uniformity within the wafer surface.

Specific examples of the developing solution having a vapor pressure of 5 kPa or less at 20°C include a ketone-based solvent such as 1-octanone, 2-octanone, 1-nonanone, 2-nonanone, 4-heptanone, 2-hexanone, diisobutyl ketone, cyclohexanone, methylcyclohexanone, phenylacetone, and methyl isobutyl ketone; an ester-based solvent such as butyl acetate, amyl acetate, propylene glycol monomethyl ether acetate, ethylene glycol monoethyl ether acetate, diethylene glycol monobutyl ether acetate, diethylene glycol monoethyl ether acetate, ethyl-3-ethoxy propionate, 3-methoxy butyl acetate, 3-methyl-3-methoxy butyl acetate, butyl formate, propyl formate, ethyl lactate, butyl lactate, and propyl lactate; an alcohol-based solvent such as n-propyl alcohol, isopropyl alcohol, n-butyl alcohol, sec-butyl alcohol, tert-butyl alcohol, isobutyl alcohol, n-hexyl alcohol, 4-methyl-2-pentanol, n-heptyl alcohol, n-octyl alcohol, and n-decanol; a glycol-based solvent such as ethylene glycol, diethylene glycol, and triethylene glycol; a glycol ether-based solvent such as ethylene glycol monomethyl ether, propylene glycol monomethyl ether, ethylene glycol monoethyl ether, propylene glycol monoethyl ether, diethylene glycol monomethyl ether, triethylene glycol monoethyl ether, and methoxymethyl butanol; an ether-based solvent such as tetrahydrofuran; an amide-based solvent such as N-methyl-2-pyrrolidone, N,N-dimethylacetamide, and N,N-dimethylformamide; an aromatic hydrocarbon-based solvent such as toluene and xylene; and an aliphatic hydrocarbon-based solvent such as octane and decane.

Specific examples of the developing solution having a vapor pressure of 2 kPa or less at 20°C include a ketone-based solvent such as 1-octanone, 2-octanone, 1-nonanone, 2-nonanone, 4-heptanone, 2-hexanone, diisobutyl ketone, cyclohexanone, methylcyclohexanone, and phenylacetone; an ester-based solvent such as butyl acetate, amyl acetate, propylene glycol monomethyl ether acetate, ethylene glycol monoethyl ether acetate, diethylene glycol monobutyl ether acetate, diethylene glycol monoethyl ether acetate, ethyl-3-ethoxy propionate, 3-methoxy butyl acetate, 3-methyl-3-methoxy butyl acetate, ethyl lactate, butyl lactate, and propyl lactate; an alcohol-based solvent such as n-butyl alcohol, sec-butyl alcohol, tert-butyl alcohol, isobutyl alcohol, n-hexyl alcohol, 4-methyl-2-pentanol, n-heptyl alcohol, n-octyl alcohol, and n-decanol; a glycol-based solvent such as ethylene glycol, diethylene glycol, and triethylene glycol; a glycol ether-based solvent such as ethylene glycol monomethyl ether, propylene glycol monomethyl ether, ethylene glycol monoethyl ether, propylene glycol monoethyl ether, diethylene glycol monomethyl ether, triethylene glycol monoethyl ether, and methoxymethyl butanol; an amide-based solvent such as N-methyl-2-pyrrolidone, N,N-dimethylacetamide, and N,N-dimethylformamide; an aromatic hydrocarbon-based solvent such as xylene; and an aliphatic hydrocarbon-based solvent such as octane and decane.

To the developing solution, a surfactant can be added in an appropriate amount, if required. The surfactant is not particularly limited but, for example, an ionic or nonionic fluorine-based and/or silicon-based surfactant can be used. Examples of the fluorine-based and/or silicon-based surfactant can include the surfactants described in Japanese Patent Laid-Open Nos. 62-36663, 61-226746, 61-226745, 62-170950, 63-34540, 7-230165, 8-62834, 9-54432, and 9-5988, and U.S. Pat. Nos. 5,405,720, 5,360,692, 5,529,881, 5,296,330, 5,436,098, 5,576,143, 5,294,511, and 5,824,451. The surfactant is preferably a nonionic surfactant. The nonionic surfactant is not particularly limited, but is preferably a fluorine-based surfactant or a silicon-based surfactant.

The amount of the surfactant used is usually 0.001 to 5% by mass based on the total amount of the developing solution, preferably 0.005 to 2% by mass, and further preferably 0.01 to 0.5% by mass.

The development method is, for example, a method for dipping a substrate in a bath filled with a developing solution for a fixed time (dipping method), a method for raising a developing solution on a substrate surface by the effect of a surface tension and keeping it still for a fixed time, thereby conducting the development (puddle method), a method for spraying a developing solution on a substrate surface (spraying method), and a method for continuously ejecting a developing solution on a substrate rotating at a constant speed while scanning a developing solution ejecting nozzle at a constant rate (dynamic dispense method), or the like may be applied. The time for conducting the pattern development is not particularly limited, but is preferably 10 seconds to 90 seconds.

After the step of conducting development, a step of stopping the development by the replacement with another solvent may be practiced.

A step of rinsing the resist film with a rinsing solution containing an organic solvent is preferably provided after the development.

The rinsing solution used in the rinsing step after development is not particularly limited as long as the rinsing solution does not dissolve the resist pattern cured by crosslinking. A solution containing a general organic solvent or water may be used as the rinsing solution. As the foregoing rinsing solution, a rinsing solution containing at least one kind of organic solvent selected from a hydrocarbon-based solvent, a ketone-based solvent, an ester-based solvent, an alcohol-based solvent, an amide-based solvent, and an ether-based solvent is preferably used. More preferably, after development, a step of rinsing the film by using a rinsing solution containing at least one kind of organic solvent selected from the group consisting of a ketone-based solvent, an ester-based solvent, an alcohol-based solvent and an amide-based solvent is conducted. Still preferably, after development, a step of rinsing the film by using a rinsing solution containing an alcohol-based solvent or an ester-based solvent is conducted. Still more preferably, after development, a step of rinsing the film by using a rinsing solution containing a monohydric alcohol is conducted. Particularly preferably, after development, a step of rinsing the film by using a rinsing solution containing a monohydric alcohol having 5 or more carbon atoms is conducted. The time for rinsing the pattern is not particularly limited, but is preferably 10 seconds to 90 seconds.

Herein, examples of the monohydric alcohol used in the rinsing step after development include a linear, branched or cyclic monohydric alcohol. Specific examples which can be used in the rinsing step include 1-butanol, 2-butanol, 3-methyl-1-butanol, tert-butyl alcohol, 1-pentanol, 2-pentanol, 1-hexanol, 4-methyl-2-pentanol, 1-heptanol, 1-octanol, 2-hexanol, cyclopentanol, 2-heptanol, 2-octanol, 3-hexanol, 3-heptanol, 3-octanol, and 4-octanol or the like. Particularly preferable examples of monohydric alcohol having 5 or more carbon atoms include 1-hexanol, 2-hexanol, 4-methyl-2-pentanol, 1-pentanol, and 3-methyl-1-butanol or the like.

A plurality of these components may be mixed, or the component may be used by mixing the component with an organic solvent other than those described above.

The water content ratio in the rinsing solution is preferably 10% by mass or less, more preferably 5% by mass or less, and still more preferably 3% by mass or less. By setting the water content ratio in the rinsing solution to 10% by mass or less, there is a tendency that better development characteristics can be obtained.

The vapor pressure at 20°C of the rinsing solution used after development is preferably 0.05 kPa or more and 5 kPa or less, more preferably 0.1 kPa or more and 5 kPa or less, and still more preferably 0.12 kPa or more and 3 kPa or less. When the vapor pressure of the rinsing solution is 0.05 kPa or more and 5 kPa or less, there is a tendency that the temperature uniformity in the wafer surface is enhanced and moreover, swelling due to permeation of the rinsing solution is further inhibited; and as a result, the dimensional uniformity in the wafer surface is further improved.

The rinsing solution may also be used after adding an appropriate amount of a surfactant to the rinsing solution.

In the rinsing step, the wafer after development is rinsed using the above organic solvent-containing rinsing solution. The method for rinsing treatment is not particularly limited. However, for example, a method for continuously ejecting a rinsing solution on a substrate spinning at a constant speed (spin coating method), a method for dipping a substrate in a bath filled with a rinsing solution for a fixed time (dipping method), and a method for spraying a rinsing solution on a substrate surface (spraying method), or the like can be applied. Above all, it is preferable to conduct the rinsing treatment by the spin coating method and after the rinsing, spin the substrate at a rotational speed of 2,000 rpm to 4,000 rpm, to remove the rinsing solution from the substrate surface.

After forming the resist pattern, a pattern wiring substrate is obtained by etching. Etching can be conducted by a publicly known method such as dry etching using plasma gas, and wet etching with an alkaline solution, a cupric chloride solution, and a ferric chloride solution or the like.

After forming the resist pattern, plating can also be conducted. Examples of the plating method include copper plating, solder plating, nickel plating, and gold plating.

The remaining resist pattern after etching can be peeled by an organic solvent. Examples of the organic solvent include PGMEA (propylene glycol monomethyl ether acetate), PGME (propylene glycol monomethyl ether), and EL (ethyl lactate). Examples of the peeling method include a dipping method and a spraying method. A wiring substrate having a resist pattern formed thereon may be a multilayer wiring substrate, and may have a small diameter through hole.

In the present embodiment, the wiring substrate can also be formed by a method for forming a resist pattern, then depositing a metal in vacuum, and subsequently dissolving the resist pattern in a solution, i.e., a liftoff method.

### [Film forming composition for lithography for underlayer film purpose]

The film forming composition for lithography for underlayer film purposes (hereinafter, also referred to as an "underlayer film forming material") of the present embodiment contains at least one substance selected from the group consisting of the compound represented by the above formula (0) and the resin obtained with the compound represented by the above formula (0) as a monomer. In the present embodiment, the content of the substance in the underlayer film forming material is preferably 1 to 100% by mass, more preferably 10 to 100% by mass, still more preferably 50 to 100% by mass, and particularly preferably 100% by mass, in the total mass of the solid components from the viewpoint of coatability and quality stability.

The underlayer film forming material of the present embodiment is applicable to a wet process and is excellent in heat resistance and etching resistance. Furthermore, the underlayer film forming material of the present embodiment employs the above substances and can therefore form an underlayer film that is prevented from deteriorating during high temperature baking and is also excellent in etching resistance against oxygen plasma etching or the like. Moreover, the underlayer film forming material of the present embodiment is also excellent in adhesiveness to a resist layer and can therefore produce an excellent resist pattern. The underlayer film forming material of the present embodiment may contain an already known underlayer film forming material for lithography or the like, within the range not deteriorating the effect of the present invention.

### [Solvent]

The underlayer film forming material of the present embodiment may contain a solvent. A publicly known solvent can be arbitrarily used as the solvent in the underlayer film forming material as long as at least the above substances dissolve.

Specific examples of the solvent include, but not particularly limited to: ketone-based solvents such as acetone, methyl ethyl ketone, methyl isobutyl ketone, and cyclohexanone; cellosolve-based solvents such as propylene glycol monomethyl ether and propylene glycol monomethyl ether acetate; ester-based solvents such as ethyl lactate, methyl acetate, ethyl acetate, butyl acetate, isoamyl acetate, methyl methoxypropionate, and methyl hydroxyisobutyrate; alcohol-based solvents such as methanol, ethanol, isopropanol, and 1-ethoxy-2-propanol; and aromatic hydrocarbons such as toluene, xylene, and anisole. These solvents can be used alone as one kind or used in combination of two or more kinds.

Among the above solvents, cyclohexanone, propylene glycol monomethyl ether, propylene glycol monomethyl ether acetate, ethyl lactate, methyl hydroxyisobutyrate, or anisole is particularly preferable from the viewpoint of safety.

The content of the solvent is not particularly limited and is preferably 100 to 10000 parts by mass per 100 parts by mass in total of the solid components, more preferably 200 to 5000 parts by mass, and still more preferably 200 to 1000 parts by mass, from the viewpoint of solubility and film formation.

### [Crosslinking agent]

The underlayer film forming material of the present embodiment may contain a crosslinking agent, if required, from the viewpoint of, for example, suppressing intermixing. The crosslinking agent is not particularly limited, but a crosslinking agent described in, for example, International Publication No. WO 2013/024779 can be used.

Specific examples of the crosslinking agent that may be used in the present embodiment include, but not particularly limited to, phenol compounds, epoxy compounds, cyanate compounds, amino compounds, benzoxazine compounds, acrylate compounds, melamine compounds, guanamine compounds, glycoluril compounds, urea compounds, isocyanate compounds, and azide compounds. These crosslinking agents can be used alone as one kind or can be used in combination of two or more kinds. Among them, a benzoxazine compound, an epoxy compound or a cyanate compound is preferable, and a benzoxazine compound is more preferable from the viewpoint of improvement in etching resistance.

As the above phenol compound, a publicly known compound can be used. Examples of phenols include phenol as well as alkylphenols such as cresols and xylenols, polyhydric phenols such as hydroquinone, polycyclic phenols such as naphthols and naphthalenediols, bisphenols such as bisphenol A and bisphenol F, and polyfunctional phenol compounds such as phenol novolac and phenol aralkyl resins. Among them, an aralkyl-based phenol resin is preferred from the viewpoint of heat resistance and solubility.

As the above epoxy compound, a publicly known compound can be used and is selected from among compounds having two or more epoxy groups in one molecule. Examples thereof include epoxidation products of dihydric phenols such as bisphenol A, bisphenol F, 3,3',5,5'-tetramethyl-bisphenol F, bisphenol S, fluorene bisphenol, 2,2'-biphenol, 3,3',5,5'-tetramethyl-4,4'-dihydroxybiphenol, resorcin, and naphthalenediols, epoxidation products of trihydric or higher phenols such as tris-(4-hydroxyphenyl)methane, 1,1,2,2-tetrakis(4-hydroxyphenyl)ethane, tris(2,3-epoxypropyl) isocyanurate, trimethylolmethane triglycidyl ether, trimethylolpropane triglycidyl ether, triethylolethane triglycidyl ether, phenol novolac, and o-cresol novolac, epoxidation products of co-condensed resins of dicyclopentadiene and phenols, epoxidation products of phenol aralkyl resins synthesized from phenols and paraxylylene dichloride, epoxidation products of biphenyl aralkyl-based phenol resins synthesized from phenols and bischloromethylbiphenyl, and epoxidation products of naphthol aralkyl resins synthesized from naphthols and paraxylylene dichloride. These epoxy resins may be used alone or in combination of two or more kinds. An epoxy resin that is in a solid state at normal temperature, such as an epoxy resin obtained from a phenol aralkyl resin or a biphenyl aralkyl resin is preferable from the viewpoint of heat resistance and solubility.

The above cyanate compound is not particularly limited as long as the compound has two or more cyanate groups in one molecule, and a publicly known compound can be used. In the present embodiment, preferable examples of the cyanate compound include cyanate compounds having a structure where hydroxy groups of a compound having two or more hydroxy groups in one molecule are replaced with cyanate groups. Also, the cyanate compound preferably has an aromatic group, and a structure where a cyanate group is directly bonded to an aromatic group can be preferably used. Examples of such a cyanate compound include cyanate compounds having a structure where hydroxy groups of bisphenol A, bisphenol F, bisphenol M, bisphenol P, bisphenol E, a phenol novolac resin, a cresol novolac resin, a dicyclopentadiene novolac resin, tetramethylbisphenol F, a bisphenol A novolac resin, brominated bisphenol A, a brominated phenol novolac resin, trifunctional phenol, tetrafunctional phenol, naphthalene-based phenol, biphenyl-based phenol, a phenol aralkyl resin, a biphenyl aralkyl resin, a naphthol aralkyl resin, a dicyclopentadiene aralkyl resin, alicyclic phenol, phosphorus-containing phenol, or the like are replaced with cyanate groups. These cyanate compounds may be used alone or in arbitrary combination of two or more kinds. Also, the above cyanate compound may be in any form of a monomer, an oligomer and a resin.

Examples of the above amino compound include m-phenylenediamine, p-phenylenediamine, 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,4'-diaminodiphenylsulfone, 3,3'-diaminodiphenylsulfone, 4,4'-diaminodiphenyl sulfide, 3,4'-diaminodiphenyl sulfide, 3,3'-diaminodiphenyl sulfide, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, bis[4-(3-aminophenoxy)phenyl] ether, 9,9-bis(4-aminophenyl)fluorene, 9,9-bis(4-amino-3-chlorophenyl)fluorene, 9,9-bis(4-amino-3-fluorophenyl)fluorene, O-tolidine, m-tolidine, 4,4'-diaminobenzanilide, 2,2'-bis(trifluoromethyl)-4,4'-diaminobiphenyl, 4-aminophenyl-4-aminobenzoate, and 2-(4-aminophenyl)-6-aminobenzoxazole. Further examples thereof include aromatic amines such as 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,3'-diaminodiphenylsulfone, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, and bis[4-(3-aminophenoxy)phenyl] ether, alicyclic amines such as diaminocyclohexane, diaminodicyclohexylmethane, dimethyl-diaminodicyclohexylmethane, tetramethyl-diaminodicyclohexylmethane, diaminodicyclohexylpropane, diaminobicyclo[2.2.1]heptane, bis(aminomethyl)-bicyclo[2.2.1]heptane, 3(4),8(9)-bis(aminomethyl)tricyclo[5.2.1.02,6]decane, 1,3-bisaminomethylcyclohexane, and isophoronediamine, and aliphatic amines such as ethylenediamine, hexamethylenediamine, octamethylenediamine, decamethylenediamine, diethylenetriamine, and triethylenetetramine.

Examples of the above benzoxazine compound include P-d-based benzoxazines obtained from difunctional diamines and monofunctional phenols, and F-a-based benzoxazines obtained from monofunctional diamines and difunctional phenols.

Specific examples of the above melamine compound include hexamethylolmelamine, hexamethoxymethylmelamine, compounds obtained by methoxymethylation of 1 to 6 methylol groups of hexamethylolmelamine, or mixtures thereof, hexamethoxyethylmelamine, hexaacyloxymethylmelamine, and compounds obtained by acyloxymethylation of 1 to 6 methylol groups of hexamethylolmelamine, or mixtures thereof.

Specific examples of the above guanamine compound include tetramethylolguanamine, tetramethoxymethylguanamine, compounds obtained by methoxymethylation of 1 to 4 methylol groups of tetramethylolguanamine, or mixtures thereof, tetramethoxyethylguanamine, tetraacyloxyguanamine, and compounds obtained by acyloxymethylation of 1 to 4 methylol groups of tetramethylolguanamine, or mixtures thereof.

Specific examples of the above glycoluril compound include tetramethylolglycoluril, tetramethoxyglycoluril, tetramethoxymethylglycoluril, compounds obtained by methoxymethylation of 1 to 4 methylol groups of tetramethylolglycoluril, or mixtures thereof, and compounds obtained by acyloxymethylation of 1 to 4 methylol groups of tetramethylolglycoluril, or mixtures thereof.

Specific examples of the above urea compound include tetramethylolurea, tetramethoxymethylurea, compounds obtained by methoxymethylation of 1 to 4 methylol groups of tetramethylolurea, or mixtures thereof, and tetramethoxyethylurea.

In the present embodiment, a crosslinking agent having at least one allyl group may be used from the viewpoint of improvement in crosslinkability. Specific examples of the crosslinking agent having at least one allyl group include, but not limited to, allylphenols such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide, and bis(3-allyl-4-hydroxyphenyl) ether, allyl cyanates such as 2,2-bis(3-allyl-4-cyanatophenyl)propane, 1,1,1,3,3,3-hexafluoro-2,2-bis(3-allyl-4-cyanatophenyl)propane, bis(3-allyl-4-cyanatophenyl)sulfone, bis(3-allyl-4-cyanatophenyl) sulfide, and bis(3-allyl-4-cyanatophenyl) ether, diallyl phthalate, diallyl isophthalate, diallyl terephthalate, triallyl isocyanurate, trimethylolpropane diallyl ether, and pentaerythritol allyl ether. These crosslinking agents may be alone, or may be a mixture of two or more kinds. Among them, an allylphenol such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide, or bis(3-allyl-4-hydroxyphenyl) ether is preferable.

The content of the crosslinking agent in the underlayer film forming material is not particularly limited and is preferably 0.1 to 50% by mass of the total mass of the solid components, more preferably 5 to 50% by mass, and still more preferably 10 to 40% by mass. By setting the content of the crosslinking agent to the above range, a mixing event with a resist layer tends to be prevented. Also, an antireflection effect is enhanced, and film formability after crosslinking tends to be enhanced.

### [Crosslinking promoting agent]

In the underlayer film forming material of the present embodiment, if required, a crosslinking promoting agent for accelerating crosslinking and curing reaction can be used.

The crosslinking promoting agent is not particularly limited as long as the crosslinking promoting agent accelerates crosslinking or curing reaction, and examples thereof include amines, imidazoles, organic phosphines, and Lewis acids. These crosslinking promoting agents can be used alone as one kind or can be used in combination of two or more kinds. Among them, an imidazole or an organic phosphine is preferable, and an imidazole is more preferable from the viewpoint of decrease in crosslinking temperature.

Examples of the crosslinking promoting agent include, but not limited to, tertiary amines such as 1,8-diazabicyclo(5,4,0)undecene-7, triethylenediamine, benzyldimethylamine, triethanolamine, dimethylaminoethanol, and tris(dimethylaminomethyl)phenol, imidazoles such as 2-methylimidazole, 2-phenylimidazole, 2-ethyl-4-methylimidazole, 2-phenyl-4-methylimidazole, 2-heptadecylimidazole, and 2,4,5-triphenylimidazole, organic phosphines such as tributylphosphine, methyldiphenylphosphine, triphenylphosphine, diphenylphosphine, and phenylphosphine, tetra substituted phosphonium-tetra substituted borates such as tetraphenylphosphonium-tetraphenyl borate, tetraphenylphosphonium-ethyltriphenyl borate, and tetrabutylphosphonium-tetrabutyl borate, and tetraphenylboron salts such as 2-ethyl-4-methylimidazole-tetraphenyl borate and N-methylmorpholine-tetraphenyl borate.

The content of the crosslinking promoting agent is usually preferably 0.1 to 10% by mass of the total mass of the solid components, and is more preferably 0.1 to 5 parts by mass, and still more preferably 0.1 to 3% by mass, from the viewpoint of easy control and cost efficiency.

### [Radical polymerization initiator]

The underlayer film forming material of the present embodiment can contain, if required, a radical polymerization initiator. The radical polymerization initiator may be a photopolymerization initiator that initiates radical polymerization by light, or may be a thermal polymerization initiator that initiates radical polymerization by heat. The radical polymerization initiator can be at least one selected from the group consisting of a ketone-based photopolymerization initiator, an organic peroxide-based polymerization initiator and an azo-based polymerization initiator.

Such a radical polymerization initiator is not particularly limited, and a radical polymerization initiator conventionally used can be arbitrarily adopted. Examples thereof include ketone-based photopolymerization initiators such as 1-hydroxy cyclohexyl phenyl ketone, benzyl dimethyl ketal, 2-hydroxy-2-methyl-1-phenylpropan-1-one, 1-[4-(2-hydroxyethoxy)-phenyl]-2-hydroxy-2-methyl-1-propan-1-one, 2-hydroxy-1-{4-[4-(2-hydroxy-2-methylpropionyl)-benzyl]phenyl}-2-methylpropan-1-one, 2,4,6-trimethylbenzoyl-diphenyl-phosphine oxide, and bis(2,4,6-trimethylbenzoyl)-phenylphosphine oxide, and organic peroxide-based polymerization initiators such as methyl ethyl ketone peroxide, cyclohexanone peroxide, methylcyclohexanone peroxide, methyl acetoacetate peroxide, acetyl acetate peroxide, 1,1-bis(t-hexylperoxy)-3,3,5-trimethylcyclohexane, 1,1-bis(t-hexylperoxy)-cyclohexane, 1,1-bis(t-butylperoxy)-3,3,5-trimethylcyclohexane, 1,1-bis(t-butylperoxy)-2-methylcyclohexane, 1,1-bis(t-butylperoxy)-cyclohexane, 1,1-bis(t-butylperoxy)cyclododecane, 1,1-bis(t-butylperoxy)butane, 2,2-bis(4,4-di-t-butylperoxycyclohexyl)propane, p-menthane hydroperoxide, diisopropylbenzene hydroperoxide, 1,1,3,3-tetramethylbutyl hydroperoxide, cumene hydroperoxide, t-hexyl hydroperoxide, t-butyl hydroperoxide, α,α'-bis(t-butylperoxy)diisopropylbenzene, dicumyl peroxide, 2,5-dimethyl-2,5-bis(t-butylperoxy)hexane, t-butylcumyl peroxide, di-t-butyl peroxide, 2,5-dimethyl-2,5-bis(t-butylperoxy)hexyne-3, isobutyryl peroxide, 3,5,5-trimethylhexanoyl peroxide, octanoyl peroxide, lauroyl peroxide, stearoyl peroxide, succinic acid peroxide, m-toluoyl benzoyl peroxide, benzoyl peroxide, di-n-propyl peroxydicarbonate, diisopropyl peroxydicarbonate, bis(4-t-butylcyclohexyl)peroxydicarbonate, di-2-ethoxyethyl peroxydicarbonate, di-2-ethoxyhexyl peroxydicarbonate, di-3-methoxybutyl peroxydicarbonate, di-s-butyl peroxydicarbonate, di(3-methyl-3-methoxybutyl) peroxydicarbonate, α,α'-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexanoate, 1-cyclohexyl-1-methylethyl peroxy-2-ethylhexanoate, t-hexyl peroxy-2-ethylhexanoate, t-butyl peroxy-2-ethylhexanoate, t-hexyl peroxyisopropylmonocarbonate, t-butyl peroxyisobutyrate, t-butyl peroxymalate, t-butyl peroxy-3,5,5-trimethylhexanoate, t-butyl peroxylaurate, t-butyl peroxyisopropylmonocarbonate, t-butyl peroxy-2-ethylhexylmonocarbonate, t-butyl peroxyacetate, t-butyl peroxy-m-toluylbenzoate, t-butyl peroxybenzoate, bis(t-butylperoxy) isophthalate, 2,5-dimethyl-2,5-bis(m-toluylperoxy)hexane, t-hexyl peroxybenzoate, 2,5-dimethyl-2,5-bis(benzoylperoxy)hexane, t-butyl peroxyallylmonocarbonate, t-butyltrimethylsilyl peroxide, 3,3',4,4'-tetra(t-butylperoxycarbonyl)benzophenone, and 2,3-dimethyl-2,3-diphenylbutane.

Further examples thereof include azo-based polymerization initiators such as 2-phenylazo-4-methoxy-2,4-dimethylvaleronitrile, 1-[(1-cyano-1-methylethyl)azo]formamide, 1,1'-azobis(cyclohexane-1-carbonitrile), 2,2'-azobis(2-methylbutyronitrile), 2,2'-azobisisobutyronitrile, 2,2'-azobis(2,4-dimethylvaleronitrile), 2,2'-azobis(2-methylpropionamidine)dihydrochloride, 2,2'-azobis(2-methyl-N-phenylpropionamidine)dihydrochloride, 2,2'-azobis[N-(4-chlorophenyl)-2-methylpropionamidine]dihydride chloride, 2,2'-azobis[N-(4-hydrophenyl)-2-methylpropionamidine]dihydrochloride, 2,2'-azobis[2-methyl-N-(phenylmethyl)propionamidine] dihydrochloride, 2,2'-azobis[2-methyl-N-(2-propenyl)propionamidine]dihydrochloride, 2,2'-azobis[N-(2-hydroxyethyl)-2-methylpropionamidine]dihydrochloride, 2,2'-azobis[2-(5-methyl-2-imidazolin-2-yl)propane] dihydrochloride, 2,2'-azobis[2-(2-imidazolin-2-yl)propane] dihydrochloride, 2,2'-azobis[2-(4,5,6,7-tetrahydro-1H-1,3-diazepin-2-yl)propane]dihydrochloride, 2,2'-azobis[2-(3,4,5,6-tetrahydropyrimidin-2-yl)propane] dihydrochloride, 2,2'-azobis[2-(5-hydroxy-3,4,5,6-tetrahydropyrimidin-2-yl)propane]dihydrochloride, 2,2'-azobis[2-[1-(2-hydroxyethyl)-2-imidazolin-2-yl]propane] dihydrochloride, 2,2'-azobis[2-(2-imidazolin-2-yl)propane], 2,2'-azobis[2-methyl-N-[1,1-bis(hydroxymethyl)-2-hydroxyethyl]propionamide], 2,2'-azobis[2-methyl-N-[1,1-bis(hydroxymethyl)ethyl]propionamide], 2,2'-azobis[2-methyl-N-(2-hydroxyethyl)propionamide], 2,2'-azobis(2-methylpropionamide), 2,2'-azobis(2,4,4-trimethylpentane), 2,2'-azobis(2-methylpropane), dimethyl-2,2-azobis(2-methylpropionate), 4,4'-azobis(4-cyanopentanoic acid), and 2,2'-azobis[2-(hydroxymethyl)propionitrile]. As the radical polymerization initiator of the present embodiment, one kind thereof may be used alone, or two or more kinds may be used in combination. Alternatively, the radical polymerization initiator of the present embodiment may be used in further combination with an additional publicly known polymerization initiator.

The content of the radical polymerization initiator can be a stoichiometrically necessary amount and is preferably 0.05 to 25% by mass of the total mass of the solid components, and more preferably 0.1 to 10% by mass. When the content of the radical polymerization initiator is 0.05% by mass or more, there is a tendency that curing can be prevented from being insufficient. On the other hand, when the content of the radical polymerization initiator is 25% by mass or less, there is a tendency that the long term storage stability of the underlayer film forming material at room temperature can be prevented from being impaired.

### [Acid generating agent]

The underlayer film forming material of the present embodiment may contain an acid generating agent, if required, from the viewpoint of, for example, further accelerating crosslinking reaction by heat. An acid generating agent that generates an acid by thermal decomposition, an acid generating agent that generates an acid by light irradiation, and the like are known, any of which can be used. For example, an acid generating agent described in International Publication No. WO 2013/024779 can be used.

The content of the acid generating agent in the underlayer film forming material is not particularly limited and is preferably 0.1 to 50% by mass of the total mass of the solid components, and more preferably 0.5 to 40% by mass. By setting the content of the acid generating agent to the above preferable range, crosslinking reaction tends to be enhanced by an increased amount of an acid generated. Also, a mixing event with a resist layer tends to be prevented.

### [Basic compound]

The underlayer film forming material of the present embodiment may contain a basic compound from the viewpoint of, for example, improving storage stability.

The basic compound plays a role as a quencher against acids in order to prevent crosslinking reaction from proceeding due to a trace amount of an acid generated by the acid generating agent. Examples of such a basic compound include, but not particularly limited to, those described in International Publication No. WO 2013/024779.

The content of the basic compound in the underlayer film forming material is not particularly limited and is preferably 0.001 to 2% by mass of the total mass of the solid components, and more preferably 0.01 to 1% by mass. By setting the content of the basic compound to the above preferable range, storage stability tends to be enhanced without excessively deteriorating crosslinking reaction.

### [Further additive agent]

The underlayer film forming material according to the present embodiment may also contain an additional resin and/or compound for the purpose of conferring thermosetting or light curing properties or controlling absorbance. Examples of such an additional resin and/or compound include, but not particularly limited to, naphthol resin, xylene resin naphthol-modified resin, phenol-modified resin of naphthalene resin, polyhydroxystyrene, dicyclopentadiene resin, resins containing (meth)acrylate, dimethacrylate, trimethacrylate, tetramethacrylate, a naphthalene ring such as vinylnaphthalene or polyacenaphthylene, a biphenyl ring such as phenanthrenequinone or fluorene, or a heterocyclic ring having a heteroatom such as thiophene or indene, and resins containing no aromatic ring; and resins or compounds containing an alicyclic structure, such as rosin-based resin, cyclodextrin, adamantine(poly)ol, tricyclodecane(poly)ol, and derivatives thereof. The underlayer film forming material according to the present embodiment may further contain a publicly known additive agent. Examples of the publicly known additive agent include, but not limited to, thermal and/or light curing catalysts, polymerization inhibitors, flame retardants, fillers, coupling agents, thermosetting resins, light curable resins, dyes, pigments, thickeners, lubricants, antifoaming agents, leveling agents, ultraviolet absorbers, surfactants, colorants, and nonionic surfactants.

### [Underlayer film for lithography and multilayer resist pattern formation method]

The underlayer film for lithography of the present embodiment is formed from the underlayer film forming material described above.

The resist pattern formation method of the present embodiment includes the steps of: forming an underlayer film on a substrate using the above composition; forming at least one photoresist layer on the underlayer film; and then irradiating a predetermined region of the photoresist layer with radiation for development. More specifically, the resist pattern formation method of the present embodiment has the steps of: forming an underlayer film on a substrate using the underlayer film forming material of the present embodiment (step (A-1)); forming at least one photoresist layer on the underlayer film (step (A-2)); and irradiating a predetermined region of the photoresist layer with radiation for development after the step (A-2) (step (A-3)).

The circuit pattern formation method of the present embodiment includes the steps of:
forming an underlayer film on a substrate using the above composition, forming an intermediate layer film on the underlayer film using a resist intermediate layer film material, and forming at least one photoresist layer on the intermediate layer film;
irradiating a predetermined region of the photoresist layer with radiation for development, thereby forming a resist pattern; and
etching the intermediate layer film with the resist pattern as a mask, etching the underlayer film with the obtained intermediate layer film pattern as an etching mask, and etching the substrate with the obtained underlayer film pattern as an etching mask, thereby forming a pattern on the substrate.

In more detail, forming an underlayer film on a substrate using the underlayer film forming material of the present embodiment (step (B-1)); forming an intermediate layer film on the underlayer film using a resist intermediate layer film material containing a silicon atom (step (B-2)); forming at least one photoresist layer on the intermediate layer film (step (B-3)); after the step (B-3), irradiating a predetermined region of the photoresist layer with radiation for development, thereby forming a resist pattern (step (B-4)); and after the step (B-4), etching the intermediate layer film with the resist pattern as a mask, etching the underlayer film with the obtained intermediate layer film pattern as an etching mask, and etching the substrate with the obtained underlayer film pattern as an etching mask, thereby forming a pattern on the substrate (step (B-5)).

The underlayer film for lithography of the present embodiment is not particularly limited by its formation method as long as it is formed from the underlayer film forming material of the present embodiment. A publicly known approach can be applied thereto. The underlayer film for lithography of the present embodiment can be formed by, for example, applying the underlayer film forming material of the present embodiment onto a substrate by a publicly known coating method or printing method such as spin coating or screen printing, and then removing an organic solvent by volatilization or the like, followed by crosslinking and curing by a publicly known method. Examples of the crosslinking method include approaches such as thermosetting and light curing.

It is preferable to perform baking in the formation of the underlayer film, for preventing a mixing event with an upper layer resist while accelerating crosslinking reaction. In this case, the baking temperature is not particularly limited and is preferably in the range of 80 to 450°C, and more preferably 200 to 400°C. The baking time is not particularly limited and is preferably in the range of 10 to 300 seconds. The thickness of the underlayer film can be arbitrarily selected according to required performance and is not particularly limited, but is usually preferably about 30 to 20000 nm, and more preferably 50 to 15000 nm.

After preparing the underlayer film, it is preferable to prepare a silicon-containing resist layer or a usual single-layer resist made of hydrocarbon thereon in the case of a two-layer process, and to prepare a silicon-containing intermediate layer thereon and further a silicon-free single-layer resist layer thereon in the case of a three-layer process. In this case, a publicly known photoresist material can be used for forming this resist layer.

After preparing the underlayer film on the substrate, in the case of a two-layer process, a silicon-containing resist layer or a usual single-layer resist made of hydrocarbon can be prepared on the underlayer film. In the case of a three-layer process, a silicon-containing intermediate layer can be prepared on the underlayer film, and a silicon-free single-layer resist layer can be further prepared on the silicon-containing intermediate layer. In these cases, a publicly known photoresist material can be arbitrarily selected and used for forming the resist layer, without particular limitations.

For the silicon-containing resist material for a two-layer process, a silicon atom-containing polymer such as a polysilsesquioxane derivative or a vinylsilane derivative is used as a base polymer, and a positive type photoresist material further containing an organic solvent, an acid generating agent, and if required, a basic compound or the like is preferably used, from the viewpoint of oxygen gas etching resistance. Herein, a publicly known polymer that is used in this kind of resist material can be used as the silicon atom-containing polymer.

A polysilsesquioxane-based intermediate layer is preferably used as the silicon-containing intermediate layer for a three-layer process. By imparting effects as an antireflection film to the intermediate layer, there is a tendency that reflection can be effectively suppressed. For example, use of a material containing a large amount of an aromatic group and having high substrate etching resistance as the underlayer film in a process for exposure at 193 nm tends to increase a k value and enhance substrate reflection. However, the intermediate layer suppresses the reflection so that the substrate reflection can be 0.5% or less. The intermediate layer having such an antireflection effect is not limited, and polysilsesquioxane that crosslinks by an acid or heat in which a light absorbing group having a phenyl group or a silicon-silicon bond is introduced is preferably used for exposure at 193 nm.

Alternatively, an intermediate layer formed by chemical vapour deposition (CVD) may be used. The intermediate layer highly effective as an antireflection film prepared by CVD is not limited, and, for example, a SiON film is known. In general, the formation of an intermediate layer by a wet process such as spin coating or screen printing is more convenient and more advantageous in cost, as compared with CVD. The upper layer resist for a three-layer process may be positive type or negative type, and the same as a single-layer resist generally used can be used.

The underlayer film according to the present embodiment can also be used as an antireflection film for usual single-layer resists or an underlying material for suppression of pattern collapse. The underlayer film is excellent in etching resistance for an underlying process and can be expected to also function as a hard mask for an underlying process.

In the case of forming a resist layer from the above photoresist material, a wet process such as spin coating or screen printing is preferably used, as in the case of forming the above underlayer film. After coating with the resist material by spin coating or the like, prebaking is generally performed. This prebaking is preferably performed at 80 to 180°C in the range of 10 to 300 seconds. Then, exposure, post-exposure baking (PEB), and development can be performed according to a conventional method to obtain a resist pattern. The thickness of the resist film is not particularly limited and is generally preferably 30 to 500 nm, and more preferably 50 to 400 nm.

The exposure light can be arbitrarily selected and used according to the photoresist material to be used. General examples thereof can include a high energy ray having a wavelength of 300 nm or less, specifically, excimer laser of 248 nm, 193 nm, or 157 nm, soft x-ray of 3 to 20 nm, electron beam, and X-ray.

In a resist pattern formed by the above method, pattern collapse is suppressed by the underlayer film. Therefore, use of the underlayer film according to the present embodiment can produce a finer pattern and can reduce an exposure amount necessary for obtaining the resist pattern.

Next, etching is performed with the obtained resist pattern as a mask. Gas etching is preferably used as the etching of the underlayer film in a two-layer process. The gas etching is preferably etching using oxygen gas. In addition to oxygen gas, an inert gas such as He or Ar, or CO, CO₂, NH₃, SO₂, N₂, NO₂, or H₂ gas may be added. Alternatively, the gas etching may be performed with CO, CO₂, NH₃, N₂, NO₂, or H₂ gas without the use of oxygen gas. Particularly, the latter gas is preferably used for side wall protection in order to prevent the undercut of pattern side walls.

On the other hand, gas etching is also preferably used as the etching of the intermediate layer in a three-layer process. The same gas etching as described in the above two-layer process is applicable. Particularly, it is preferable to process the intermediate layer in a three-layer process by using chlorofluorocarbon-based gas and using the resist pattern as a mask. Then, as mentioned above, for example, the underlayer film can be processed by oxygen gas etching with the intermediate layer pattern as a mask.

Herein, in the case of forming an inorganic hard mask intermediate layer film as the intermediate layer, a silicon oxide film, a silicon nitride film, or a silicon oxynitride film (SiON film) is formed by CVD, ALD, or the like. A method for forming the nitride film is not limited, and, for example, a method described in Japanese Patent Laid-Open No. 2002-334869 (Patent Literature 6) or WO2004/066377 (Patent Literature 7) can be used. Although a photoresist film can be formed directly on such an intermediate layer film, an organic antireflection film (BARC) may be formed on the intermediate layer film by spin coating and a photoresist film may be formed thereon.

A polysilsesquioxane-based intermediate layer is preferably used as the intermediate layer. By imparting effects as an antireflection film to the resist intermediate layer film, there is a tendency that reflection can be effectively suppressed. A specific material for the polysilsesquioxane-based intermediate layer is not limited, and, for example, a material described in Japanese Patent Laid-Open No. 2007-226170 (Patent Literature 8) or Japanese Patent Laid-Open No. 2007-226204 (Patent Literature 9) can be used.

The subsequent etching of the substrate can also be performed by a conventional method. For example, the substrate made of SiO₂ or SiN can be etched mainly using chlorofluorocarbon-based gas, and the substrate made of p-Si, Al, or W can be etched mainly using chlorine- or bromine-based gas. In the case of etching the substrate with chlorofluorocarbon-based gas, the silicon-containing resist of the two-layer resist process or the silicon-containing intermediate layer of the three-layer process is peeled at the same time with substrate processing. On the other hand, in the case of etching the substrate with chlorine- or bromine-based gas, the silicon-containing resist layer or the silicon-containing intermediate layer is separately peeled and in general, peeled by dry etching using chlorofluorocarbon-based gas after substrate processing.

A feature of the underlayer film according to the present embodiment is that it is excellent in etching resistance of the substrates. The substrate can be arbitrarily selected from publicly known ones and used and is not particularly limited. Examples thereof include Si, α-Si, p-Si, SiO₂, SiN, SiON, W, TiN, and Al. The substrate may be a laminate having a film to be processed (substrate to be processed) on a base material (support). Examples of such a film to be processed include various low-k films such as Si, SiO₂, SiON, SiN, p-Si, α-Si, W, W-Si, Al, Cu, and Al-Si, and stopper films thereof. A material different from that for the base material (support) is generally used. The thickness of the substrate to be processed or the film to be processed is not particularly limited and is generally preferably about 50 to 10,000 nm, and more preferably 75 to 5,000 nm.

### [Resist permanent film]

The above composition can also be used to prepare a resist permanent film. The resist permanent film prepared by coating with the above composition is suitable as a permanent film that also remains in a final product, if required, after formation of a resist pattern. Specific examples of the permanent film include, in relation to semiconductor devices, solder resists, package materials, underfill materials, package adhesive layers for circuit elements and the like, and adhesive layers between integrated circuit elements and circuit substrates, and in relation to thin displays, thin film transistor protecting films, liquid crystal color filter protecting films, black matrixes, and spacers. Particularly, the permanent film made of the above composition is excellent in heat resistance and humidity resistance and furthermore, also has the excellent advantage that contamination by sublimable components is reduced. Particularly, for a display material, a material that achieves all of high sensitivity, high heat resistance, and hygroscopic reliability with reduced deterioration in image quality due to significant contamination can be obtained.

In the case of using the above composition for resist permanent film purposes, a curing agent as well as, if required, various additive agents such as other resins, a surfactant, a dye, a filler, a crosslinking agent, and a dissolution promoting agent can be added and dissolved in an organic solvent to prepare a composition for resist permanent films.

The above film forming composition for lithography or composition for resist permanent films can be prepared by adding each of the above components and mixing them using a stirrer or the like. When the above composition for resist underlayer films or composition for resist permanent films contains a filler or a pigment, it can be prepared by dispersion or mixing using a dispersion apparatus such as a dissolver, a homogenizer, and a three-roll mill.

### Examples

The present embodiment will be described in more detail with reference to synthesis working examples, synthesis examples, examples and comparative examples below. However, the present embodiment is not limited to these examples by any means.

### (Carbon concentration and oxygen concentration)

The carbon concentration and the oxygen concentration (% by mass) were measured by organic elemental analysis using the following apparatus.

Apparatus: CHN Coder MT-6 (manufactured by Yaic. Yanaco)

### (Molecular weight)

The molecular weight of a compound was measured by LC-MS analysis using Acquity UPLC/MALDI-Synapt HDMS manufactured by Waters Corp.

Also, the weight average molecular weight (Mw), number average molecular weight (Mn), and dispersibility (Mw/Mn) in terms of polystyrene were determined by gel permeation chromatography (GPC) analysis under the following conditions.

Apparatus: Shodex GPC-101 model (manufactured by Showa Denko K.K.)
Column: KF-80M × 3
Eluent: 1 mL/min THF
Temperature: 40°C

### (Solubility)

A compound was dissolved at 3% by weight and 10% by mass in propylene glycol monomethyl ether (PGME), cyclohexanone (CHN), ethyl lactate (EL), methyl amyl ketone (MAK) or tetramethylurea (TMU) at 23°C. Then, results about the solution obtained 1 week later were evaluated according to the following criteria.

Evaluation S: No precipitate was visually confirmed from 10% by mass of the compound in any of the solvents.

Evaluation A: No precipitate was visually confirmed from 3% by weight of the compound in any of the solvents.

Evaluation C: Precipitates were visually confirmed in all of the solvents.

### [Structure of compound]

The structure of a compound was confirmed by 1H-NMR measurement using "Advance 600II spectrometer" manufactured by Bruker Corp. under the following conditions.

Frequency: 400 MHz
Solvent: d6-DMSO
Internal standard: TMS
Measurement temperature: 23°C

### <Synthesis Working Example 1> Synthesis of BiF-1

To a container (internal capacity: 300 mL) equipped with a stirrer, a condenser tube, and a burette, after 13 g (69.0 mmol) of 2,2'-biphenol (a reagent manufactured by Tokyo Chemical Industry Co., Ltd.) was melted at 120°C, 0.27 g of sulfuric acid was added, and 2.7 g (13.8 mmol) of 4-acetylbiphenyl (a reagent manufactured by Sigma-Aldrich) was added, and the contents were reacted by being stirred at 120°C for 6 hours to obtain a reaction solution. Next, 100 mL of N-methyl-2-pyrrolidone (manufactured by Kanto Chemical Co., Inc.) and 50 mL of pure water were added to the reaction solution, followed by extraction with ethyl acetate. Next, the mixture was separated until neutral by the addition of pure water, and then concentrated to obtain a solution.

The obtained solution was separated by column chromatography to obtain 1.0 g of the objective compound (BiF-1) represented by the following formula (BiF-1).

As a result of measuring the molecular weight of the obtained compound (BiF-1) by the above method, it was 550.

The thermal decomposition temperature was 330°C, and the glass transition temperature was 102°C. Thus, it was able to be confirmed that the compound has high heat resistance. Since no melting point was observed, it was able to be confirmed that the compound is highly amorphous and is easily molded by spin coating.

The following peaks were found by NMR measurement performed on the obtained compound (BiF-1) under the above measurement conditions, and the compound was confirmed to have a chemical structure of the following formula (BiF-1). δ (ppm) 9.20 (4H, O-H), 6.77-7.66 (23H, Ph-H), 2.09 (3H, C-H)

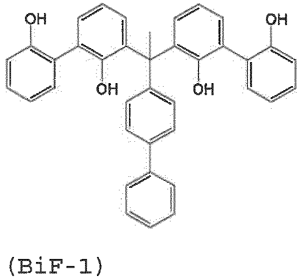

### <Synthesis Working Example 2> Synthesis of BiF-3

The same operations as above were performed except that 4-acetylbiphenyl was changed to 4-biphenylaldehyde to obtain 2.0 g of the objective compound (BiF-3) represented by the following formula (BiF-3).

As a result of measuring the molecular weight of the obtained compound (BiF-3) by the above method, it was 536.

The thermal decomposition temperature was 275°C, and the glass transition temperature was 102°C. Thus, it was able to be confirmed that the compound has high heat resistance. Since no melting point was observed, it was able to be confirmed that the compound is highly amorphous and is easily molded by spin coating.

The following peaks were found by NMR measurement performed on the obtained compound (BiF-3) under the above measurement conditions, and the compound was confirmed to have a chemical structure of the following formula (BiF-3). δ (ppm) 9.15 (4H, O-H), 6.78-7.64 (23H, Ph-H), 5.47 (1H, C-H)

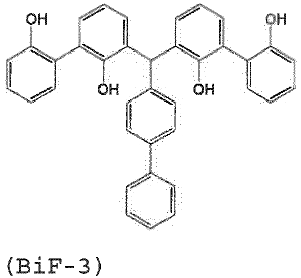

### (Synthesis Working Examples 3 to 7)

The same operations as in Synthesis Working Example 1 were performed except that 4-acetylbiphenyl which were raw materials in Synthesis Working Example 1 were changed as shown in Raw material 2 of Table 1 below to obtain each target compound.

Each compound was identified by 1H-NMR (Table 2).

**[Table 1]**

| Synthesis Example | Raw material 1 | Raw material 2 | Product |
|---|---|---|---|
| 3 | 2,2'-Biphenol | 4'-Cyclohexylacetophenone | BiF-2 |
| 4 | 2,2'-Biphenol | Isobutylbenzaldehyde | BiF-4 |
| 5 | 2,2'-Biphenol | n-Propylbenzaldehyde | BiF-5 |
| 6 | 2,2'-Biphenol | 4-Hydroxybenzaldehyde | BiF-6 |
| 7 | 2,2'-Biphenol | 4-Cyclohexylbenzaldehyde | BiF-7 |

**[Table 2]**

| Synthesis Example | Compound name | 1H-NMR |
|---|---|---|
| 3 | BiF-2 | *δ* (ppm) 9.1(4H, O-H), 6.7∼7.6(18H, Ph-H), 5.47(1H, C-H), 2.3(3H, C-H3), 1.4∼1.9(10H, C-H2) |
| 4 | BiF-4 | *δ* (ppm) 9.1(4H, O-H), 6.9∼7.8(18H, Ph-H), 6.6(1H, C-H), 2.3(6H, C -H3), 1.4∼1.9(3H, -CH2-CH) |
| 5 | BiF-5 | *δ* (ppm)9.2∼9.7(4H, O-H), 6.8∼7.8(18H, Ph-H), 6.6(1H, C-H), 2.4 (3H, C-H3), 1.4∼1.8(3H, -CH2-CH2) |
| 6 | BiF-6 | *δ* (ppm) 9.4∼9.7(5H, O-H), 6.8∼7.8(18H, Ph-H), 6.6(1H, C-H) |
| 7 | BiF-7 | *δ* (ppm) 9.1(4H, O-H), 6.8∼7.6(18H, Ph-H), 6.6(1H, C-H), 1.4∼1.9 (10H, C-H2) |

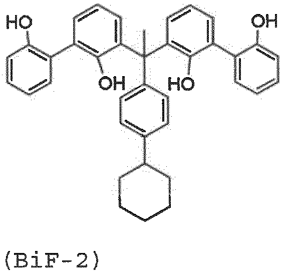

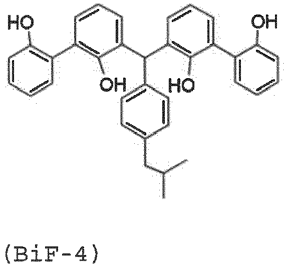

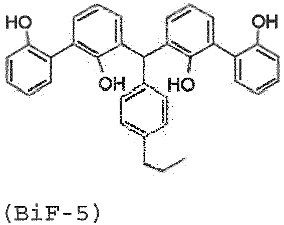

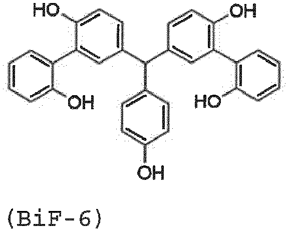

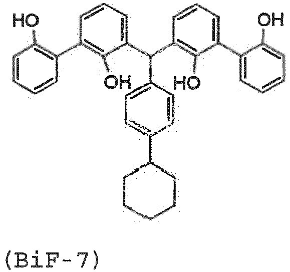

### (Synthesis Working Example 8) Synthesis of resin (R1-BiF-1)

A four necked flask (internal capacity: 1 L) equipped with a Dimroth condenser tube, a thermometer, and a stirring blade and having a detachable bottom was prepared. To this four necked flask, 39.6 g (70 mmol) of the compound (BiF-1) obtained in Synthesis Working Example 1 (manufactured by Mitsubishi Gas Chemical Company, Inc.), 21.0 g (280 mmol as formaldehyde) of 40% by mass of an aqueous formalin solution (manufactured by Mitsubishi Gas Chemical Company, Inc.), and 0.97 mL of 98% by mass of sulfuric acid (manufactured by Kanto Chemical Co., Inc.) were added in a nitrogen stream, and the mixture was reacted for 7 hours while refluxed at 100°C at normal pressure. Subsequently, 180.0 g of orthoxylene (special grade reagent manufactured by Wako Pure Chemical Industries, Ltd.) was added as a diluting solvent to the reaction solution, and the mixture was left to stand still, followed by removal of an aqueous phase as a lower phase. Neutralization and washing with water were further performed, and orthoxylene was distilled off under reduced pressure to obtain 31.6 g of a brown solid resin (R1-BiF-1).

The molecular weight of the obtained resin (R1-BiF-1) was Mn: 1985, Mw: 3420, Mw/Mn: 1.72.

### (Synthesis Working Example 9) Synthesis of resin (R2-BiF-1)

A four necked flask (internal capacity: 1 L) equipped with a Dimroth condenser tube, a thermometer, and a stirring blade and having a detachable bottom was prepared. To this four necked flask, 39.6 g (70 mmol) of the compound (BiF-1) obtained in Synthesis Working Example 1 (manufactured by Mitsubishi Gas Chemical Company, Inc.), 50.9 g (280 mmol) of 4-biphenylaldehyde (manufactured by Mitsubishi Gas Chemical Company, Inc.), 100 mL of anisole (manufactured by Kanto Chemical Co., Inc.), and 10 mL of oxalic acid dihydrate (manufactured by Kanto Chemical Co., Inc.) were added in a nitrogen stream, and the mixture was reacted for 7 hours while refluxed at 100°C at normal pressure. Subsequently, 180.0 g of orthoxylene (special grade reagent manufactured by Wako Pure Chemical Industries, Ltd.) was added as a diluting solvent to the reaction solution, and the mixture was left to stand still, followed by removal of an aqueous phase as a lower phase. Neutralization and washing with water were further performed, and the solvent in the organic phase and unreacted 4-biphenylaldehyde were distilled off under reduced pressure to obtain 34.7 g of a brown solid resin (R2-BiF-1) .

The molecular weight of the obtained resin (R2-BiF-1) was Mn: 1612, Mw: 3040, Mw/Mn: 1.89.

### (Synthesis Comparative Example 1)

A four necked flask (internal capacity: 10 L) equipped with a Dimroth condenser tube, a thermometer, and a stirring blade and having a detachable bottom was prepared. To this four necked flask, 1.09 kg (7 mol) of 1,5-dimethylnaphthalene (manufactured by Mitsubishi Gas Chemical Company, Inc.), 2.1 kg (28 mol as formaldehyde) of 40% by mass of an aqueous formalin solution (manufactured by Mitsubishi Gas Chemical Company, Inc.), and 0.97 mL of 98% by mass of sulfuric acid (manufactured by Kanto Chemical Co., Inc.) were added in a nitrogen stream, and the mixture was reacted for 7 hours while refluxed at 100°C at normal pressure. Subsequently, 1.8 kg of ethylbenzene (special grade reagent manufactured by Wako Pure Chemical Industries, Ltd.) was added as a diluting solvent to the reaction solution, and the mixture was left to stand still, followed by removal of an aqueous phase as a lower phase. Neutralization and washing with water were further performed, and ethylbenzene and unreacted 1,5-dimethylnaphthalene were distilled off under reduced pressure to obtain 1.25 kg of a light brown solid dimethylnaphthalene formaldehyde resin.

The molecular weight of the obtained dimethylnaphthalene formaldehyde was Mn: 562.

Subsequently, a four necked flask (internal capacity: 0.5 L) equipped with a Dimroth condenser tube, a thermometer, and a stirring blade was prepared. To this four necked flask, 100 g (0.51 mol) of the dimethylnaphthalene formaldehyde resin obtained as above, and 0.05 g of p-toluenesulfonic acid were added in a nitrogen stream, and the temperature was raised to 190°C at which the mixture was then heated for 2 hours, followed by stirring. Subsequently, 52.0 g (0.36 mol) of 1-naphthol was added thereto, and the temperature was further raised to 220°C at which the mixture was reacted for 2 hours. After solvent dilution, neutralization and washing with water were performed, and the solvent was removed under reduced pressure to obtain 126.1 g of a black-brown solid modified resin (CR-1).

The obtained resin (CR-1) had Mn: 885, Mw: 2220, and Mw/Mn: 4.17.

### (Examples 1 to 9 and Comparative Example 1)

The above compounds and resins of Synthesis Working Examples 1 to 9, and CR-1 were evaluated for their respective solubility. The results are shown in Table 3.

Underlayer film forming materials for lithography were each prepared according to the composition shown in Table 3. Next, a silicon substrate was spin coated with each of these underlayer film forming materials for lithography, and then baked at 240°C for 60 seconds and further at 400°C for 120 seconds to prepare each underlayer film with a film thickness of 200 nm. The following acid generating agent, crosslinking agent, and organic solvent were used.

Acid generating agent: di-tertiary butyl diphenyliodonium nonafluoromethanesulfonate (DTDPI) manufactured by Midori Kagaku Co., Ltd.
Crosslinking agent: NIKALAC MX270 (NIKALAC) (Sanwa Chemical Co., Ltd.)
Organic solvent: propylene glycol monomethyl ether acetate (PGMEA)

### [Etching resistance]

Etching test was further conducted under conditions shown below to evaluate etching resistance. The evaluation results are shown in Tables.

### [Etching test]

Etching apparatus: RIE-10NR manufactured by Samco International, Inc.
Output: 50 W
Pressure: 20 Pa
Time: 2 min
Etching gas
Ar gas flow rate:CF₄ gas flow rate:O₂ gas flow rate = 50:5:5 (sccm)

The evaluation of etching resistance was conducted by the following procedures.

First, an underlayer film of novolac was prepared under the same conditions as in Example 1 except that novolac (PSM4357 manufactured by Gunei Chemical Industry Co., Ltd.) was used instead of the compound (BiF-1). Then, this underlayer film of novolac was subjected to the above etching test, and the etching rate was measured.

Next, underlayer films of Examples 1 to 9, Examples 1A to 9A and Comparative Example 1 were subjected to the above etching test in the same way as above, and the etching rate was measured.

Then, the etching resistance was evaluated according to the following evaluation criteria on the basis of the etching rate of the underlayer film of novolac.

### [Evaluation criteria]

A: The etching rate was less than -10% as compared with the underlayer film of novolac.
B: The etching rate was -10% to +5% as compared with the underlayer film of novolac.
C: The etching rate was more than +5% as compared with the underlayer film of novolac.

**[Table 3]**

| | Underlayer film forming material | Solubility | Composition for underlayer film forming material for lithography | | | | Etching resistance |
|---|---|---|---|---|---|---|---|
| | | | Underlayer film forming material (parts by mass) | Solvent PGMEA (parts by mass) | Acid generating agent DTDPI (parts by mass) | Crosslinking agent NIKALAC (parts by mass) | |
| Example 1 | BiF-1 | A | 10 | 190 | 0.5 | 0.5 | A |
| Example 1A | BiF-1 | A | 10 | 190 | 0 | 0 | B |
| Example 2 | BiF-3 | A | 10 | 190 | 0.5 | 0.5 | A |
| Example 2A | BiF-3 | A | 10 | 190 | 0 | 0 | B |
| Example 3 | BiF-2 | A | 10 | 190 | 0.5 | 0.5 | A |
| Example 3A | BiF-2 | A | 10 | 190 | 0 | 0 | B |
| Example 4 | BiF-4 | A | 10 | 190 | 0.5 | 0.5 | A |
| Example 4A | BiF-4 | A | 10 | 190 | 0 | 0 | B |
| Example 5 | BiF-5 | A | 10 | 190 | 0.5 | 0.5 | A |
| Example 5A | BiF-5 | A | 10 | 190 | 0 | 0 | B |
| Example 6 | BiF-6 | A | 10 | 190 | 0.5 | 0.5 | A |
| Example 6A | BiF-6 | A | 10 | 190 | 0 | 0 | B |
| Example 7 | BiF-7 | A | 10 | 190 | 0.5 | 0.5 | A |
| Example 7A | BiF-7 | A | 10 | 190 | 0 | 0 | B |
| Example 8 | R1-BiF-1 | A | 10 | 190 | 0.5 | 0.5 | A |
| Example 8A | R1-BiF-1 | A | 10 | 190 | 0 | 0 | B |
| Example 9 | R2-BiF-1 | A | 10 | 190 | 0.5 | 0.5 | A |
| Example 9A | R2-BiF-1 | A | 10 | 190 | 0 | 0 | B |
| Comparative Example 1 | CR-1 | A | 10 | 190 | 0.5 | 0.5 | C |

### (Examples 10 and 11)

Next, a SiO₂ substrate with a film thickness of 300 nm was coated with each solution of the underlayer film forming material for lithography containing BiF-1 or BiF-3 obtained in Example 1 or 2, and baked at 240°C for 60 seconds and further at 400°C for 120 seconds to prepare each underlayer film with a film thickness of 70 nm. This underlayer film was coated with a resist solution for ArF and baked at 130°C for 60 seconds to form a photoresist layer with a film thickness of 140 nm. The ArF resist solution used was prepared by containing 5 parts by mass of a compound of the formula (11) given below, 1 part by mass of triphenylsulfonium nonafluoromethanesulfonate, 2 parts by mass of tributylamine, and 92 parts by mass of PGMEA.

For the compound of the formula (11), 4.15 g of 2-methyl-2-methacryloyloxyadamantane, 3.00 g of methacryloyloxy-γ-butyrolactone, 2.08 g of 3-hydroxy-1-adamantyl methacrylate, and 0.38 g of azobisisobutyronitrile were dissolved in 80 mL of tetrahydrofuran to prepare a reaction solution. This reaction solution was polymerized for 22 hours with the reaction temperature kept at 63°C in a nitrogen atmosphere. Then, the reaction solution was added dropwise into 400 mL of n-hexane. The product resin thus obtained was solidified and purified, and the resulting white powder was filtered and dried overnight at 40°C under reduced pressure to obtain a compound represented by the following formula.

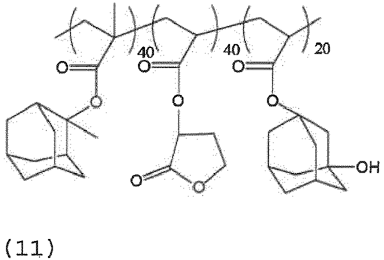

wherein "40", "40", and "20" represent the ratio of each constituent unit and do not represent a block copolymer.

Subsequently, the photoresist layer was exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a positive type resist pattern.

The shape and defects of the obtained resist patterns of 55 nmL/S (1:1) and 80 nmL/S (1:1) were observed using an electron microscope manufactured by Hitachi Ltd. (S-4800).

The shapes of the resist patterns after development were evaluated as "goodness" when having good rectangularity without pattern collapse, and as "poorness" if this was not the case. The smallest line width having good rectangularity without pattern collapse as a result of this observation was used as an index for "resolution" evaluation. The smallest electron beam energy quantity capable of lithographing good pattern shapes was used as an index for "sensitivity" evaluation.

The evaluation results are shown in Table 4.

### (Comparative Example 2)

The same operations as in Example 3 were performed except that no underlayer film was formed so that a photoresist layer was formed directly on a SiO₂ substrate to obtain a positive type resist pattern. The results are shown in Table 4.

**[Table 4]**

| | Underlayer film forming material | Resolution (nmL/S) | Sensitivity (µC/cm²) | Resist pattern shape after development |
|---|---|---|---|---|
| Example 10 | BiF-1 | 45 | 10 | Good |
| Example 11 | BiF-3 | 45 | 10 | Good |
| Comparative Example 2 | None | 80 | 26 | Poor |

As is evident from Table 1, Examples 1 and 2 using BiF-1 and BiF-3 were at least confirmed to be good in terms of all of heat resistance, solubility and etching resistance, as compared with Comparative Example 1. On the other hand, Comparative Example 1 using CR-1 (phenol-modified dimethylnaphthaleneformaldehyde resin) resulted in poor etching resistance.

In Examples 10 and 11, the resist pattern shape after development was confirmed to be good without any defect. These examples were further confirmed to be significantly superior in both resolution and sensitivity to Comparative Example 2 in which underlayer film formation was omitted.

In addition, the difference in the resist pattern shapes after development also demonstrated that the underlayer film forming materials for lithography used in Examples 1 and 2 have good adhesiveness to a resist material.

### <Examples 12 and 13>

A SiO₂ substrate with a film thickness of 300 nm was coated with the solution of the underlayer film forming material for lithography of each of Examples 1 to 2, and baked at 240°C for 60 seconds and further at 400°C for 120 seconds to form each underlayer film with a film thickness of 80 nm. This underlayer film was coated with a silicon-containing intermediate layer material and baked at 200°C for 60 seconds to form an intermediate layer film with a film thickness of 35 nm. This intermediate layer film was further coated with the above resist solution for ArF and baked at 130°C for 60 seconds to form a photoresist layer with a film thickness of 150 nm. The silicon-containing intermediate layer material used was the silicon atom-containing polymer obtained as described below.

In 200 g of tetrahydrofuran (THF) and 100 g of pure water, 16.6 g of 3-carboxylpropyltrimethoxysilane, 7.9 g of phenyltrimethoxysilane, and 14.4 g of 3-hydroxypropyltrimethoxysilane were dissolved, and the temperature of the solution was adjusted to 35°C. 5 g of oxalic acid was dropped thereto, and then, the temperature of the mixture was raised to 80°C to perform the condensation reaction of silanol. Next, an aqueous layer was separated by the addition of 200 g of diethyl ether, and the organic liquid layer was washed twice with ultrapure water, and 200 g of propylene glycol monomethyl ether acetate (PGMEA) was added thereto. While the temperature of the solution was raised to 60°C by heating, THF and diethyl ether water were removed under reduced pressure to obtain a silicon atom-containing polymer.

Subsequently, the photoresist layer was mask exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a 55 nm L/S (1:1) positive type resist pattern.

Then, the silicon-containing intermediate layer film (SOG) was dry etched with the obtained resist pattern as a mask using RIE-10NR manufactured by Samco International, Inc. Subsequently, dry etching of the underlayer film with the obtained silicon-containing intermediate layer film pattern as a mask and dry etching of the SiO₂ film with the obtained underlayer film pattern as a mask were performed in order.

### Respective etching conditions are as shown below.

Conditions for etching of resist intermediate layer film with resist pattern
Output: 50 W
Pressure: 20 Pa
Time: 1 min
Etching gas
Ar gas flow rate:CF₄ gas flow rate:O₂ gas flow rate = 50:8:2 (sccm)
Conditions for etching of resist underlayer film with resist intermediate film pattern
Output: 50 W
Pressure: 20 Pa
Time: 2 min
Etching gas
Ar gas flow rate:CF₄ gas flow rate:O₂ gas flow rate = 50:5:5 (seem)
Conditions for etching of SiO₂ film with resist underlayer film pattern
Output: 50 W
Pressure: 20 Pa
Time: 2 min
Etching gas
Ar gas flow rate: C₅F₁₂ gas flow rate: C₂F₆ gas flow rate:O₂ gas flow rate = 50:4:3:1 (sccm)

### [Evaluation]

The pattern cross section (the shape of the SiO₂ film after etching) obtained as described above was observed under an electron microscope manufactured by Hitachi, Ltd. (S-4800). As a result, it was confirmed that the shape of the SiO₂ film after etching in a multilayer resist process is a rectangular shape in Examples using the underlayer film of the present embodiment and is good without defects.

### [Examples 14 and 15]

An optical component forming composition was prepared according to the recipe shown below in Table 5 using each compound synthesized in the above synthesis examples and synthesis working examples. Among the components of the optical component forming composition in Table 5, the following acid generating agent, acid crosslinking agent, acid diffusion controlling agent, and solvent were used.

Acid generating agent: di-tertiary butyl diphenyliodonium nonafluoromethanesulfonate (DTDPI) manufactured by Midori Kagaku Co., Ltd.
Crosslinking agent: NIKALAC MX270 (NIKALAC) (Sanwa Chemical Co., Ltd.)
Organic solvent: propylene glycol monomethyl ether acetate acetate (PGMEA)

A clean silicon wafer was spin coated with the homogeneous optical component forming composition, and then prebaked (PB) in an oven of 110°C to form an optical component forming film with a thickness of 1 µm. The prepared optical component forming composition was evaluated as "A" when it formed a good film, and as "C" when the formed film had defects.

A clean silicon wafer was spin coated with the homogeneous optical component forming composition, and then prebaked (PB) in an oven of 110°C to form a film with a thickness of 1 µm. The refractive index (λ = 589.3 nm) of the film at 25°C was measured using a variable angle spectroscopic ellipsometer VASE manufactured by J. A. Woollam Co., Inc. The prepared film was evaluated as "A" when the refractive index was 1.6 or more, as "B" when the refractive index was 1.55 or more and less than 1.6, and as "C" when the refractive index was less than 1.55. Also, the film was evaluated as "A" when the transparency (λ = 632.8 nm) was 90% or more, and as "C" when the transparency was less than 90%.

**[Table 5]**

| | Underlayer film forming material | Composition for optical component forming material | | | | Evaluation | | |
|---|---|---|---|---|---|---|---|---|
| | | Underlayer film forming material (parts by mass) | Solvent PGMEA (parts by mass) | Acid generating agent DTDPI (parts by mass) | Crosslinking agent NIKALAC (parts by mass) | Film formation | Refractive index | Transmittance |
| Example 14 | BiF-1 | 10 | 190 | 0.5 | 2 | A | B | A |
| Example 15 | BiF-3 | 10 | 190 | 0.5 | 2 | A | A | A |
| Comparative Example 3 | CR-1 | 10 | 190 | 0.5 | 2 | A | C | C |

### [Examples 16 and 17]

A resist composition was prepared according to the recipe shown in Table 6 using each compound synthesized in the above synthesis examples and synthesis working examples. Among the components of the resist composition in Table 6, the following acid generating agent, acid crosslinking agent, acid diffusion controlling agent, and solvent were used.

Acid generating agent: triphenylphosphonium trifluoromethanesulfonate manufactured by Midori Kagaku Co., Ltd.
Crosslinking agent: NIKALAC MX270 manufactured by Sanwa Chemical Co., Ltd.
Acid diffusion controlling agent: trioctylamine manufactured by Tokyo Chemical Industry Co., Ltd.
Organic solvent: propylene glycol monomethyl ether (PGME) manufactured by Tokyo Chemical Industry Co., Ltd.

### [Evaluation method]

### (1) Storage stability and thin film formation of resist composition

The storage stability of the resist composition was evaluated by leaving the resist composition after preparation to stand still at 23°C and 50% RH for 3 days, and visually observing the presence or absence of precipitates. The resist composition thus left to stand still for 3 days was evaluated as A when it was a homogeneous solution without precipitates, and as C when there were precipitates. A clean silicon wafer was spin coated with the homogeneous resist composition, and then prebaked (PB) before exposure in an oven of 110°C to form a resist film with a thickness of 40 nm. The prepared resist composition was evaluated as A when it formed a good thin film, and as C when the formed film had defects.

### (2) Pattern evaluation of resist pattern

A clean silicon wafer was spin coated with the homogeneous resist composition, and then prebaked (PB) before exposure in an oven of 110°C to form a resist film with a thickness of 60 nm. The obtained resist film was irradiated with electron beams of 1:1 line and space setting with 50 nm, 40 nm and 30 nm intervals using an electron beam lithography system (ELS-7500 manufactured by ELIONIX INC.). After the irradiation, the resist film was heated at each predetermined temperature for 90 seconds, and immersed in PGME for 60 seconds for development. Subsequently, the resist film was washed with ultrapure water for 30 seconds, and dried to form a negative type resist pattern. Concerning the formed resist pattern, the line and space were observed under a scanning electron microscope (S-4800 manufactured by Hitachi High-Technologies Corporation) to evaluate the reactivity by electron beam irradiation of the resist composition.

The sensitivity was indicated by the smallest energy quantity per unit area necessary for obtaining patterns, and evaluated according to the following criteria.
A: When a pattern was obtained at less than 50 µC/cm²
C: When a pattern was obtained at 50 µC/cm² or more

As for pattern formation, the obtained pattern shape was observed under SEM (scanning electron microscope), and evaluated according to the following criteria.
A: When a rectangular pattern was obtained
B: When an almost rectangular pattern was obtained
C: When a non-rectangular pattern was obtained

**[Table 6]**

| | Compound | Composition for resist | | | | | Evaluation | | | |
|---|---|---|---|---|---|---|---|---|---|---|
| | | Compound (parts by mass) | Acid generating agent (parts by mass) | Crosslinking agent (parts by mass) | Acid diffusion controlling agent (parts by mass) | Solvent (parts by mass) | Storage stability | Thin film formation | Sensitivity | Pattern formation |
| Example 16 | BiF-1 | 1 | 0 | 0 | 0 | 50 | A | A | A | A |
| Example 17 | BiF-3 | 1 | 0 | 0 | 0 | 50 | A | A | A | A |
| Comparative Example 4 | CR-1 | 1 | 0.3 | 0.3 | 0.03 | 50 | A | A | C | C |

As mentioned above, the present embodiment is not limited to the above embodiments and examples, and changes or modifications can be arbitrarily made without departing from the spirit of the present invention.

The compound and the resin according to the present invention have high solubility in a safe solvent and have good heat resistance and etching resistance. The resist composition according to the present invention imparts a good shape to a resist pattern.

Also, the compound and the resin of the present embodiment are applicable to a wet process and can achieve a compound, a resin, and a film forming composition for lithography useful for forming a photoresist underlayer film excellent in heat resistance and etching resistance. Furthermore, this film forming composition for lithography employs the compound or the resin having high heat resistance and also high solvent solubility and having a specific structure and can therefore form a resist and an underlayer film that is prevented from deteriorating during high temperature baking and is also excellent in etching resistance against oxygen plasma etching or the like. Moreover, the underlayer film thus formed is also excellent in adhesiveness to a resist layer and can therefore form an excellent resist pattern.

Moreover, the composition of the present embodiment has high refractive index and is prevented from being stained by low temperature to high temperature treatments. Therefore, the composition is also useful as various optical component forming compositions.

Accordingly, the present invention is used in for example, electrical insulating materials, resins for resists, encapsulation resins for semiconductors, adhesives for printed circuit boards, electrical laminates mounted in electric equipment, electronic equipment, industrial equipment, and the like, matrix resins of prepregs mounted in electric equipment, electronic equipment, industrial equipment, and the like, buildup laminate materials, resins for fiber-reinforced plastics, resins for encapsulation of liquid crystal display panels, coating materials, various coating agents, adhesives, coating agents for semiconductors, resins for resists for semiconductors, resins for underlayer film formation, and in the form of a film or a sheet, and additionally, can be used widely and effectively in optical components such as plastic lenses (prism lenses, lenticular lenses, microlenses, Fresnel lenses, viewing angle control lenses, contrast improving lenses, etc.), phase difference films, films for electromagnetic wave shielding, prisms, optical fibers, solder resists for flexible printed wiring, plating resists, interlayer insulating films for multilayer printed circuit boards, and photosensitive optical waveguides.

The present application is based on Japanese Patent Application No. 2016-183026 filed in the Japan Patent Office on September 20, 2016, the contents of which are incorporated herein by reference.

### Industrial Applicability

The present invention has industrial applicability in the fields of resists for lithography, underlayer films for lithography, underlayer films for multilayer resists, and optical components.

## Claims

1. A compound represented by following formula (0): 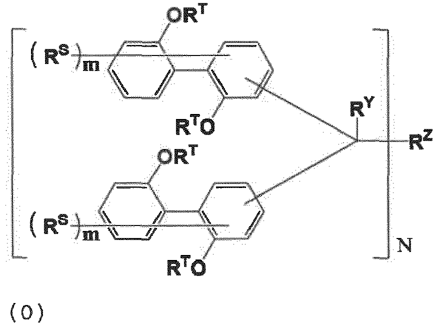
wherein R^{Y} is a hydrogen atom, an alkyl group, which has 1 to 30 carbon atoms or an aryl group, which has 6 to 30 carbon atoms;
R^{Z} is an N-valent group, which has 1 to 60 carbon atoms or a single bond;
R^{T} is a hydrogen atom;
each R^{s} is independently an alkyl group, which has 1 to 30 carbon atoms, and which optionally has a substituent, an aryl group, which has 6 to 30 carbon atoms, and which optionally has a substituent, an alkenyl group, which has 2 to 30 carbon atoms, and which optionally has a substituent, an alkoxy group, which has 1 to 30 carbon atoms, and which optionally has a substituent, a halogen atom, a nitro group, an amino group, a carboxylic acid group, a thiol group or a hydroxy group, wherein the alkyl group, the aryl group, the alkenyl group, and the alkoxy group each optionally contain an ether bond, a ketone bond, or an ester bond;
each m is independently an integer of 0 to 7; and
N is an integer of 1 to 4, wherein when N is an integer of 2 or larger, N structural formulas within the parentheses [ ] are the same or different.

2. The compound according to claim 1, wherein the compound represented by the above formula (0) is a compound represented by following formula (1): 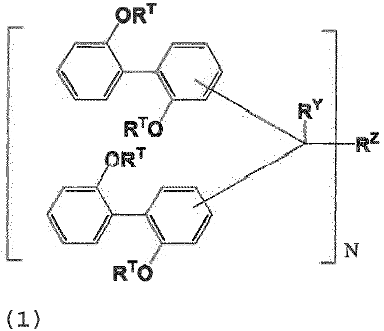 wherein R^{Y}, R^{Z}, R^{T} and N are as defined above.

3. The compound according to claim 2, wherein the compound represented by the above formula (1) is a compound represented by following formula (1-1): 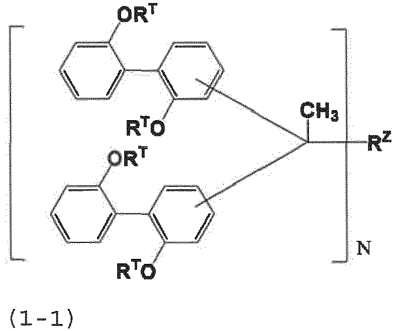 wherein R^{Z}, R^{T} and N are as defined above.

4. The compound according to claim 2, wherein the compound represented by the above formula (1) is a compound represented by following formula (1-2): 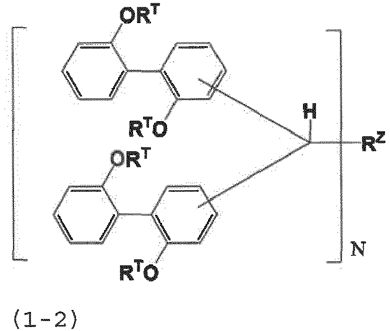 wherein R^{Y}, R^{Z}, R^{T} and N are as defined above.

5. A resin obtained with the compound according to claim 1 as a monomer.

6. The resin according to claim 5, wherein the resin has a structure represented by following formula (2): 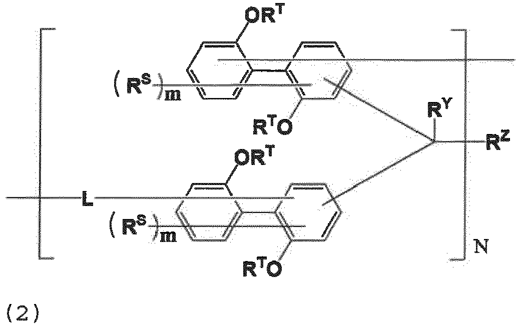
wherein R^{Y}, R^{Z}, R^{T}, R^{S}, N and m are as defined above; and
L is a linear, branched, or cyclic alkylene group, which has 1 to 30 carbon atoms, and which optionally has a substituent, an arylene group, which has 6 to 30 carbon atoms, and which optionally has a substituent, an alkoxylene group, which has 1 to 30 carbon atoms, and which optionally has a substituent or a single bond, wherein the alkylene group, the arylene group, and the alkoxylene group each optionally contain an ether bond, a ketone bond, or an ester bond.

7. The resin according to claim 6, wherein the resin represented by the above formula (2) has a structure represented by following formula (3): 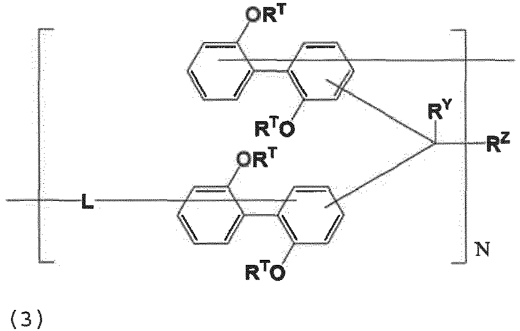 wherein R^{Y}, R^{Z}, R^{T}, N and L are as defined above.

8. A composition comprising one or more selected from the group consisting of the compound according to any one of claims 1 to 4 and the resin according to any one of claims 5 and 6.

9. The composition according to claim 8, further comprising a solvent.

10. The composition according to claim 8 or 9, further comprising an acid generating agent.

11. The composition according to any one of claims 8 to 10, further comprising a crosslinking agent.

12. The composition according to claim 11, wherein the crosslinking agent is at least one selected from the group consisting of a phenol compound, an epoxy compound, a cyanate compound, an amino compound, a benzoxazine compound, a melamine compound, a guanamine compound, a glycoluril compound, a urea compound, an isocyanate compound and an azide compound.

13. The composition according to claim 11 or 12, wherein the crosslinking agent has at least one allyl group.

14. The composition according to any one of claims 11 to 13, wherein a content of the crosslinking agent is 0.1 to 50% by mass based on a total mass of a solid component.

15. The composition according to any one of claims 11 to 14, further comprising a crosslinking promoting agent.

16. The composition according to claim 15, wherein the crosslinking promoting agent is at least one selected from the group consisting of an amine, an imidazole, an organic phosphine, and a Lewis acid.

17. The composition according to claim 15 or 16, wherein a content of the crosslinking promoting agent is 0.1 to 5% by mass based on a total mass of a solid component.

18. The composition according to any one of claims 8 to 17, further comprising a radical polymerization initiator.

19. The composition according to any one of claims 8 to 18, wherein the radical polymerization initiator is at least one selected from the group consisting of a ketone-based photopolymerization initiator, an organic peroxide-based polymerization initiator and an azo-based polymerization initiator.

20. The composition according to any one of claims 8 to 19, wherein a content of the radical polymerization initiator is 0.05 to 25% by mass based on a total mass of a solid component.

21. The composition according to any one of claims 8 to 20, wherein the composition is used in film formation for lithography.

22. The composition according to any one of claims 8 to 20, wherein the composition is used in resist permanent film formation.

23. The composition according to any one of claims 8 to 20, wherein the composition is an optical component forming composition.

24. A method for forming a resist pattern, comprising the steps of: forming a photoresist layer on a substrate using the composition according to claim 21; and then irradiating a predetermined region of the photoresist layer with radiation for development.

25. A method for forming a resist pattern, comprising the steps of: forming an underlayer film on a substrate using the composition according to claim 21; forming at least one photoresist layer on the underlayer film; and then irradiating a predetermined region of the photoresist layer with radiation for development.

26. A method for forming a circuit pattern, comprising the steps of: forming an underlayer film on a substrate using the composition according to claim 21, forming an intermediate layer film on the underlayer film using a resist intermediate layer film material, and forming at least one photoresist layer on the intermediate layer film;
irradiating a predetermined region of the photoresist layer with radiation for development, thereby forming a resist pattern; and
etching the intermediate layer film with the resist pattern as a mask, etching the underlayer film with the obtained intermediate layer film pattern as an etching mask, and etching the substrate with the obtained underlayer film pattern as an etching mask, thereby forming a pattern on the substrate.
